# NOUVEAU FASCICULE DE BREVET EUROPEEN

(11) **EP 1 277 463 B2**
(45) Date de publication et mention de la décision concernant l'opposition: **13.08.2014**
(45) Mention de la délivrance du brevet: 02.01.2008
(21) Numéro de dépôt: 02291580.5
(22) Date de dépôt: 25.06.2002
(51) Int. Cl.: A61K 8/25, A61K 8/39, A61K 8/55, A61K 8/60, A61K 8/73, A61K 8/81, A61K 8/86, A61K 8/88, C11D 1/66, C11D 1/74, C11D 3/00, A61Q 5/02, A61Q 19/10, C11D 1/34, C11D 3/12, C11D 3/22, C11D 3/37, C11D 17/00

(54) **Composition moussante à base de silice et de polymère cationique**
Schaumbildende Zusammensetzung auf der Basis von Kieselsäure und einem kationischen Polymer
Foaming composition of silica and cationic polymer

(30) Priorité: 20.07.2001 FR 0109767
(43) Date de publication de la demande: 22.01.2003
(73) Titulaire: L'Oréal, 75008 Paris (FR)
(72) Inventeur: Sebillotte-Arnaud, Laurence, 94240 l'Hay les Roses (FR); Bordeaux, Dominique, 91310 Longpont sur Orge (FR)
(74) Mandataire: Duvert, Sandra

(56) Documents cités:
- EP-A- 0 559 375
- EP-A- 0 761 205
- EP-A- 0 898 960
- EP-A1- 0 684 035
- EP-A2- 1 106 167
- WO-A-00/40208
- WO-A1-00/38621
- WO-A1-98/05633
- DD-A- 201 458
- DE-A- 19 937 917
- GB-A- 2 091 100
- US-A- 4 534 892
- US-A- 5 885 948
- US-A- 6 080 708
- PATENT ABSTRACTS OF JAPAN vol. 016, no. 261 (C-0950), 12 juin 1992 (1992-06-12) & JP 04 059716 A (NOEVIR CO LTD), 26 février 1992 (1992-02-26)

## Description

La présente invention se rapporte à une composition de nettoyage moussante rinçable comprenant une silice, un polymère cationique et/ou amphotère et un composé oxyalkyléné spécifique, et à l'utilisation de ladite composition notamment dans le domaine cosmétique ou dermatologique, comme produits de nettoyage ou de démaquillage de la peau, des yeux, du cuir chevelu et/ou des cheveux, pour traiter la peau humaine et/ou pour désinfecter la peau et/ou le cuir chevelu.

Le nettoyage de la peau est très important pour le soin du visage. Il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons.

Un moyen pour bien nettoyer la peau est d'utiliser des produits de nettoyage moussants. Les produits de nettoyage moussants actuellement disponibles dans le commerce se présentent sous forme de pains, de gels ou de crèmes moussants, et ils peuvent ou non contenir des savons (sels d'acides gras). Les produits moussants avec savons ont l'avantage de donner une mousse onctueuse ; cependant, certains consommateurs reprochent à ces produits de provoquer des tiraillements dus à leur détergence trop importante. Pour avoir un produit mieux toléré, on cherche à diminuer le taux de savons. Toutefois, le produit a alors une viscosité insuffisante.

Par ailleurs, les produits moussants sans savon sont généralement bien tolérés. Toutefois, les qualités de mousse sont moins bonnes que celles obtenues avec les produits qui contiennent des savons. Une façon d'améliorer les qualités de mousse et d'obtenir des qualités proches de celles des produits avec savon (mousse douce, dense, avec des bulles de petite taille), est d'introduire des polymères cationiques. Cependant, les polymères cationiques ont l'inconvénient de laisser un film sur la peau, ce qui se traduit par un rinçage dit filmogène ou glissant. Ce type de rinçage n'est pas apprécié par les consommateurs car il donne l'impression d'une peau mal lavée.

Ainsi, il subsiste le besoin de produits moussants rinçables, qui même sans savon, aient les propriétés requises pour des produits moussants, à savoir de bonnes qualités de la mousse obtenue, tout en présentant une bonne stabilité.

La demanderesse a découvert, de manière surprenante, d'une part que l'introduction de silice dans les compositions moussantes contenant un polymère cationique permet d'améliorer nettement le rinçage en diminuant la sensation désagréable de glissant, et ceci, quel que soit le type de polymère cationique, et d'autre part que l'incompatibilité entre les polymères cationiques et les silices pouvant conduire à des produits hétérogènes et instables, peut être surmontée grâce à la présence de composés oxyalkylénés spécifiques.

Ainsi, les produits associant silice, composé oxyalkyléné spécifique et polymère cationique ou amphotère non seulement sont stables et homogènes mais aussi ont de = bonnes propriétés, c'est-à-dire qu'ils se rincent mieux que ceux contenant des polymères seuls et qu'ils donnent à la fois une bonne qualité de mousse et de bonnes propriétés cosmétiques.

Pour atteindre cet objectif, la silice doit être présente en une quantité suffisante, cette quantité étant d'au moins 1 % en poids par rapport au poids total de la composition.

Outre les propriétés indiquées ci-dessus, certains polymères cationiques tels que le polyquaternium-7 permettent une transformation du produit lors de l'étalement sur la peau, plus rapide que les autres polymères. Cela se traduit par la formation d'un film opaque blanc, que l'on appelle « gant blanc » ou « pouvoir couvrant », très homogène et très couvrant qui ne permet plus de distinguer la couleur de la peau. Cette propriété permet au consommateur de visualiser nettement mieux et plus rapidement la zone d'application du produit, et ainsi de mieux nettoyer sa peau.

Certes, il est connu d'utiliser de la silice dans des compositions nettoyantes ou détergentes. Ainsi, par exemple, le document US-A-5,880,076 décrit une composition détergente liquide pouvant contenir des silices. Les documents EP-A-550 281 et US-A-5,389,279 citent la silice comme poudre pouvant être incorporée dans des compositions de nettoyage. Toutefois, aucun document ne décrit d'associer une quantité suffisante de silice, un composé oxyalkyléné spécifique et un polymère cationique pour obtenir une composition moussante qui, même sans savon, se rapproche très fortement des qualités de mousse d'une composition avec savon.

Ainsi, la présente demande a pour objet une composition de nettoyage comprenant, dans un milieu aqueux physiologiquement acceptable, (1) au moins un tensioactif moussant, dont la quantité va de 3 à 50% en poids de matière active par rapport au poids total de la composition, (2) au moins 1 % en poids d'au moins une silice, par rapport au poids total de la composition, (3) au moins un composé oxyalkyléné spécifique et (4) au moins un polymère choisi parmi les polymères cationiques, les polymères amphotères et leurs mélanges.

On entend ici par « milieu physiologiquement acceptable » un milieu compatible avec la peau, les muqueuses, le cuir chevelu, les yeux et/ou les cheveux. Par ailleurs, il s'agit d'un milieux aqueux, c'est-à-dire d'un milieu comportant de l'eau et de préférence une quantité d'eau d'au moins 35 % en poids, par exemple allant de 35 à 95 % en poids et mieux de 40 à 80 % en poids par rapport au poids total de la composition.

Les compositions de l'invention sont des compositions de nettoyage moussantes et rinçables. Elles se présentent sous forme d'un gel pouvant s'écouler ou non sous son propre poids, c'est-à-dire ayant une viscosité pouvant aller par exemple de 5 poises à 250 poises (0,5 à 25 Pa.s) et de préférence de 35 poises à 200 poises (3,5 à 20 Pa.s), la viscosité étant mesurée à 25°C avec un appareil de mesure Rheomat 180 à 200 s⁻¹, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités supérieures à 20 poises.

### Silice

La silice peut être choisie parmi les silices hydrophiles, les silices hydrophobes et leurs mélanges. On entend par « silice » dans la présente demande aussi bien les silices pures (hydrophiles ou hydrophobes) que les particules enrobées de silice.

La quantité de silice(s) dans la composition de l'invention, qu'il s'agisse de silice pure ou de particules enrobées de silice, doit être d'au moins 1 % en poids de matière active, pour atteindre le but de l'invention, et elle peut aller par exemple, en poids de matière active, de 1 à 15 % en poids, mieux de 2 à 10 % en poids et encore mieux de 3 à 6 % en poids par rapport au poids total de la composition.

### Silices hydrophiles

Ces silices sont de préférence amorphes et elles peuvent être d'origine pyrogénée ou d'origine précipitée. Elles peuvent se présenter sous forme pulvérulente ou en dispersion aqueuse.

Les silices hydrophiles pyrogénées sont obtenues par pyrolyse en flamme continue à 1000°C du tétrachlorure de silicium (SiCl₄) en présence d'hydrogène et d'oxygène.

Les silices précipitées sont obtenues par réaction d'un acide sur des solutions de silicates alcalins, de préférence du silicate de soude.

Selon un mode préféré de réalisation de l'invention, la silice hydrophile est choisie parmi les silices ayant une surface spécifique de 30 à 500 m²/g, une dimension moyenne de particules en nombre allant de 3 à 50 nm et une densité tassée allant de 40 à 200 et mieux de 50 à 150 g/l. Ce sont plus particulièrement les silices hydrophiles décrites dans les tableaux (1) et (2) ci-dessous, et leurs mélanges.

Ces silices sont commercialisées par la société Degussa-Hüls.

**Tableau (1)**

| Nom commercial | AEROSIL 90 | AEROSIL 130 | AEROSIL 150 | AEROSIL 200 |
|---|---|---|---|---|
| Mode d'obtention | Pyrogénation | Pyrogénation | Pyrogénation | Pyrogénation |
| Surface spécifique BET (m²/g) | 90 ± 15 | 130 ± 25 | 150 ± 15 | 200 ± 15 |
| Dimension moyenne des particules (nm) | 20 | 16 | 14 | 12 |
| Densité tassée (g/l) | Environ 80 | Environ 50 | Environ 50 | Environ 50 |
| Densité des groupes silanol (OH/m²) | 2-3 | 2-3 | 2-3 | 2-3 |
| pH à 4% dans l'eau | 3,6-4,5 | 3,6-4,5 | 3,6-4,3 | 3,6-4,3 |
| Remarque | | | | taille des agrégats : 10-30 et 200 µ |

**Tableau (2)**

| Nom commercial | AEROSIL 300 | AEROSIL 380 | AEROSIL OX 50 | SILICE FK 320 DS |
|---|---|---|---|---|
| Mode d'obtention | Pyrogénation | Pyrogénation | Pyrogénation | Précipitation |
| Surface spécifique BET (m²/g) | 300 ± 30 | 380 ±30 | 50 ± 25 | 170 ± 25 |
| Grandeur moyen des particules (nm) | 7 | 7 | 40 | 18 |
| Densité tassée (g/l) | Environ 50 | Environ 50 | Environ 130 | Environ 80 |
| Densité des groupes silanol (OH/m²) | 2-3 | 2-3 | 2-3 | - |
| pH à 4% dans l'eau | 3,6 - 4,5 | 3,6 - 4,5 | 3,8 - 4,5 | 6,3 |

On peut aussi utiliser de la silice en dispersion aqueuse, et par exemple une dispersion de silice colloïdale, telle que le produit commercialisé sous la dénomination BINDZIL 30/220® par la société Eka Chemicals, dispersion colloïdale de silice amorphe (taille : 14 nanomètres) dans l'eau (30/70).

La silice hydrophile pouvant être utilisée dans la composition de l'invention peut consister également en une particule comprenant une surface de silice, par exemple une particule recouverte totalement ou partiellement de silice, notamment d'une particule minérale recouverte totalement ou partiellement de silice, telle que les billes de silice contenant de l'oxyde de titane, commercialisées sous la dénomination TORAYCERAM S-IT® par la société Toray; les microsphères de silice-alumine contenant de l'oxyde de titane (taille : 105 µ), commercialisées sous la dénomination Z-LIGHT-SPHERE W 1012® par la société Zeelan ; les particules de silice synthétique précipitée amorphe/oxyde de titane (taille : 106-500 µ), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 µ), commercialisée sous la dénomination ACS-0050510® par la société SACI-CFPA ; le nano-oxyde de titane anatase traité alumine et silice à 40% dans l'eau (taille : 60 nm, monodisperse), commercialisé sous la dénomination MIRASUN TIW 60® par la société Rhodia Chimie CRA ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie CRA ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane: 30 nm ; épaisseur de silice: 2 nm) commercialisé sous la dénomination MAXLIGHT TS-042® par la société Nichimen Europe PLC. Ces particules peuvent également avoir des propriétés optiques dans le produit comme sur la peau. Par exemple, elles peuvent avoir un effet matifiant ou légèrement blanchissant.

On utilise de préférence comme silice hydrophile, les silices pyrogénées et en particulier celles commercialisées sous les dénominations AEROSIL 200® et AEROSIL 300® par la société Degussa-Hüls.

### Silices hydrophobes

Les silices hydrophobes amorphes d'origine pyrogénée sont obtenues à partir de silices hydrophiles. Comme décrit ci-dessus, ces dernières sont obtenues par pyrolyse en flamme continue à 1000°C du tétrachlorure de silicium (SiCl₄) en présence d'hydrogène et d'oxygène. Elles sont ensuite rendues hydrophobes par un traitement avec des silanes halogénés, des alcoxysilanes ou des silazanes. Les silices hydrophobes diffèrent des silices hydrophiles de départ, entre autres, par une densité de groupes silanols plus faible et par une adsorption de vapeur d'eau plus petite.

Selon un mode préféré de réalisation de l'invention, la silice hydrophobe est choisie parmi les silices ayant une surface spécifique de 50 à 500 m²/g, une dimension moyenne de particules en nombre allant de 3 à 50 nm et une densité tassée allant de 40 à 200 et mieux de 50 à 150 g/l. Ce sont plus particulièrement les silices hydrophobes décrites dans le tableau (3) ci-dessous, et leurs mélanges. Ces silices sont commercialisées par la société Degussa-Hüls.

**Tableau (3)**

| Nom commercial | AEROSIL R202 | AEROSIL R805 | AEROSIL R812 | AEROSIL R972 | AEROSIL R974 |
|---|---|---|---|---|---|
| Surface spécifique BET (m²/g) | 90 ± 20 | 150 ± 25 | 260 ± 30 | 110 ± 20 | 170 ± 20 |
| Dimension moyenne des particules (nm) | 14 | 12 | 7 | 16 | 12 |
| Densité tassée (g/l) | Environ 50 | Environ 50 | Environ 50 | Environ 50 | Environ 50 |
| pH à 4% dans l'eau | 4-6 | 3,5-5,5 | 5,5-7,5 | 3,6-4,3 | 3,4-4,2 |

La silice hydrophobe pouvant être utilisée dans la composition de l'invention peut consister également en une particule recouverte totalement ou partiellement de silice, notamment d'une particule minérale recouverte totalement ou partiellement de silice hydrophobe, telle que les pigments et oxydes métalliques recouverts de silice hydrophobe. Ces particules peuvent aussi avoir des propriétés optiques dans le produit comme sur la peau, par exemple elles peuvent avoir un effet matifiant ou légèrement blanchissant.

On utilise de préférence comme silice hydrophobe, celle commercialisée sous la dénomination AEROSIL R972® par la société Degussa-Hüls.

### Composés oxyalkylénés

Le composé oxyalkyléné spécifique est choisi seul ou en mélange dans le groupe consistant en le PEG-120 methyl glucose dioléate (un tel composé est commercialisé sous la dénomination GLUCAMATE DOE-120 VEGETAL TM par la société Amerchol), le tétrastéarate de pentaérythrityle oxyéthyléné (150 OE) (un tel composé est commercialisé sous la dénomination CROTHIX TM par la société Croda), le copolymère alcool stéarylique oxyéthyléné (100 OE) / polyéthylène glycol (136 OE) / hexaméthylène diisocyanate (un tel composé est commercialisé sous la dénomination SERAD FX 1100 par la société Adriss).

Le ou les composés autres oxyalkylénés qui peuvent être utilisés en complément dans la composition de l'invention peuvent comprendre des groupes d'oxyde d'éthylène (composés oxyéthylénés), des groupes d'oxyde de propylène (composés oxypropylénés) ou les deux (composés oxyéthylénés/oxypropylénés).

On peut utiliser un ou plusieurs composés oxyalkylénés, et la quantité totale de composé(s) oxyalkyléné(s) dans la composition de l'invention peut aller par exemple, en poids de matière active, de 0,1 à 20 % en poids, de préférence de 0,5 à 10 % en poids et mieux de 1 à 5 % en poids par rapport au poids total de la composition.

Les composés oxyalkylénés peuvent être choisis notamment parmi les polyéthylène glycols, les dérivés de polyéthylène glycol et/ou de polypropylène glycol, les copolymères oxyéthylénés / oxypropylénés, les dérivés alkyl ou acyl alkoxylés de polyol, les triesters de glycérol et d'acides gras oxyalkylénés et notamment oxyéthylénés, les dérivés uréthane éthoxyéthylénés modifiés par des chaînes alkyle, et leurs mélanges.

Selon un mode préféré de réalisation de l'invention, le composé oxyalkyléné complémentaire comporte au moins 10 motifs oxyéthylénés appelés aussi motifs d'oxyde d'éthylène.
1. Les polyéthylène glycols complémentaires pouvant être utilisés dans la composition de l'invention sont des polycondensats d'oxyde d'éthylène, ayant un nombre de motifs d'oxyde d'éthylène (OE) supérieur à 10. Le nombre d'oxyde d'éthylène peut aller par exemple de 10 à 50 000 et de préférence de 14 à 10 000. Comme polyéthylène glycols, on peut citer par exemple le polyéthylène glycol comportant 7 000 OE (nom CTFA : PEG-7M) comme le produit commercialisé sous la dénomination POLYOX WSR N-750® par la société Amerchol, le polyéthylène glycol comportant 75 OE (nom CTFA : PEG-75), le polyéthylène glycol comportant 20 000 OE (nom CTFA: PEG-20M) comme le produit commercialisé sous la dénomination POLYOX WSR 1105® par la société Amerchol, le polyéthylène glycol comportant 150 OE (nom CTFA : PEG-150).
2. Les dérivés de polyéthylène glycol et/ou de polypropylène glycol sont des condensats de polyéthylène glycol et/ou polypropylène glycol avec un ou plusieurs acides gras ou alcools gras. Ce sont des composés de formule (I) :

   R - (OE)ₘ - (OP)ₙ - R' (I)

   dans laquelle 0 ≤ m ≤ 300 et 0 ≤ n ≤ 300, et m+n ≥ 6, R et R' représentent indépendamment l'un de l'autre, une chaîne alkyle, acyle ou aralkyle saturée ou insaturée, linéaire ou ramifiée, hydroxylée ou non, comportant de 1 à 26 atomes de carbone et de préférence de 12 à 20 atomes de carbone, ou une chaîne aryle.
   Ces dérivés peuvent être par exemple des esters d'acides gras et de polyéthylène glycol et/ou de polypropylène glycol, et des éthers d'alcools gras et de polyéthylène glycol et/ou de polypropylène glycol.
   Comme esters d'acides gras et de polyéthylène glycol et/ou de polypropylène glycol, on peut citer par exemple le distéarate de polyéthylène glycol (150 OE) tel que le produit commercialisé sous la dénomination ATLAS G-1821® par la société Uniqema, le distéarate de polyéthylène glycol (250 OE) tel que le produit commercialisé sous la dénomination EMANON 3299R® par la société Kao, le PEG-150 dibehenate tel que le produit commercialisé sous la dénomination ETHOX PEG 6000 Dibehenate® par la société Ethox, le palmito-stéarate de polyéthylène glycol (120 OE) tel que le produit commercialisé sous la dénomination STEARATE 6000 WL 1644® par la société Gattefosse, le copolymère de polyéthylène glycol (30 OE) et d'acide 12- hydroxystéarique tel que le produit commercialisé sous la dénomination ARLACEL P135® par la société Uniqema, le stéarate de polyéthylène glycol (40 OE) tel que le produit commercialisé sous la dénomination MYRJ 52® par la société Uniqema.
   Comme exemples d'éthers de polyéthylène glycol, on peut citer notamment l'alcool cétylique oxyéthyléné (30 OE) tel que le produit commercialisé sous la dénomination NIKKOL BC-30TX® par la société Nikkol, l'alcool oléylique oxyéthyléné (15 OE) tel que le produit commercialisé sous la dénomination NIKKOL BO-15TX® par la société Nikkol, l'alcool oléylique oxyéthyléné (50 OE) tel que le produit commercialisé sous la dénomination NIKKOL BO-50® par la société Nikkol, l'alcool béhénylique oxyéthyléné (10 OE) tel que le produit commercialisé sous la dénomination MERGITAL B 10® par la société Nikkol, l'alcool béhénylique oxyéthyléné (30 OE) tel que le produit commercialisé sous la dénomination NIKKOL BB-30® par la société Nikkol, l'alcool laurylique oxyéthyléné (12 OE) tel que le produit commercialisé sous la dénomination REWOPAL 12® par la société Goldschmidt, l'alcool laurylique oxyéthyléné (23 OE) tel que le produit commercialisé sous la dénomination SIMULSOL P 23® par la société Seppic, l'alcool octyl-2 dodécylique oxyéthyléné (20 OE) tel que le produit commercialisé sous la dénomination OCTYLDODECETH-20® par la société Stearinerie Dubois, l'alcool isocétylique oxyéthyléné (20 OE) tel que le produit commercialisé sous la dénomination ARLASOLVE 200 US® par la société Uniqema, l'alcool oléylique oxyéthyléné (10 OE) tel que le produit commercialisé sous la dénomination BRIJ 97 ® par la société Uniqema, l'alcool oléylique oxyéthyléné (20 OE) tel que le produit commercialisé sous la dénomination BRIJ 98® par la société Uniqema, l'alcool stéarylique oxyéthyléné (100 OE) tel que le produit commercialisé sous la dénomination BRIJ 700® par la société Uniqema, l'alcool stéarylique oxyéthyléné (21 OE) tel que le produit commercialisé sous la dénomination BRIJ 721® par la société Uniqema.
   Comme exemples d'éthers de polyéthylène glycol/polypropylène glycol, on peut citer notamment l'alcool cétylique oxyéthyléné (20 OE) oxypropyléné (5 OP) tel que le produit commercialisé sous la dénomination PROCETYL AWS® par la société Croda, l'alcool butylique oxyéthyléné (26 OE) oxypropyléné (26 OP) tel que le produit commercialisé sous la dénomination PPG-26-BUTETH-26® par la société Goldschmidt, l'alcool butylique oxyéthyléné (26 OE) oxypropyléné (26 OP) tel que le produit commercialisé sous la dénomination VARONIC APEB® par la société Goldschmidt, le décyl-tetra-decanol oxyéthyléné (30 OE) oxypropyléné (6 OP) tel que le produit commercialisé sous la dénomination NIKKOL PEN-4630® par la société Nikkol, l'alcool laurylique oxyéthyléné (25 OE) oxypropyléné (25 OP) tel que le produit commercialisé sous la dénomination ADF-OLEILE® par la société Vevy.
3. Comme copolymères oxyéthylénés / oxypropylénés, on peut citer par exemple le copolymère statistique polyoxyéthylène / polyoxypropylène (17 OE / 6 OP) commercialisé sous la référence UCON 75-H-450® par la Société Amerchol. Les molécules comportant plus d'OE et/ou plus d'OP ne sont pas exclues.
4. Les dérivés alkyl ou acyl éthoxylés de polyol peuvent être par exemple des dérivés oxyéthylénés d'esters d'acide gras ou d'éthers d'alcool gras et de polyol tel que glycérol, sorbitol, glucose, pentaerythritol.
   Comme dérivés de ce type, on peut citer par exemple, le cocoate de glycéryle oxyéthyléné (78 OE) tel que le produit commercialisé sous la dénomination SIMULSOL CG par la société Seppic, le septa-oléate de sorbitane oxyéthyléné (40 OE) tel que le produit commercialisé sous la dénomination ARLATONE T ® par la société Uniqema, le laurate de polyglycéryle (2 moles de glycérol) oxyéthyléné (10 OE) tel que le produit commercialisé sous la dénomination HOE S 3495 ® par la société Clariant, l'isostéarate de glycéryle oxyéthyléné (60 OE) tel que le produit commercialisé sous la dénomination EMALEX GWIS-160 ® par la société SACI-CFPA, le monostéarate de glycéryle oxyéthyléné (20 OE) tel que le produit commercialisé sous la dénomination CUTINA E 24 ® par la société Cognis, le stéarate de glycéryle oxyéthyléné (200 OE) tel que le produit commercialisé sous la dénomination SIMULSOL 220 TM ® par la société Seppic.
5. Comme triesters de glycérol et d'acides gras oxyalkylénés et notamment oxyéthylénés, on peut citer par exemple l'huile d'olive oxyéthylénée (50 OE) tel que le produit commercialisé sous la dénomination CROVOL O-70® par la société Croda.
6. Comme dérivés uréthane éthoxyéthylénés modifiés par des chaînes alkyle, on peut citer par exemple ceux de formules (II) et (III) :

   R₁-NH-CO-(O-CH₂-CH₂)ₐ-[O-CO-NR₄-R₃-NR₄-CO-(O-CH₂-CH₂)ₐ]_{b}-O-CO-NH-R₂ (II)

   R₅-(O-CH₂-CH₂)ₙ-O-CO-NH-R₆-NH-CO-(O-CH₂-CH₂)ₙ-O-R₅ (III)

   dans lesquelles les radicaux R₁, R₂ et R₅ représentent un groupe alkyle comportant de 1 à 18 atomes de carbone ; R₃ et R₆ représentent un radical hydrocarboné linéaire, cyclique ou aromatique comportant de 4 à 36 atomes de carbone ; R₄ représente un atome d'hydrogène ou un radical alkyle comportant de 1 à 6 atomes de carbone, de préférence un atome d'hydrogène ; a est un nombre entier allant de 90 à 600, b est un nombre entier allant de 1 à 4, et n est un nombre entier allant de 10 à 300.

On peut citer par exemple les polymères hydrosolubles obtenus par réaction d'addition de diisocyanates (HMDI : Hexamethylene diisocyanate) sur des diols (polyethers, polyesters) et terminés par des groupements hydrophobes provenant d'alcools gras éthoxylés ou éthoxylés/propoxylés.

On peut citer aussi par exemple les produits commercialisés sous les dénominations ACRYSOL 44 (ou ACULYN 44) et ACRYSOL 46 (ou ACULYN 46) (nom CTFA: PEG-150/DECYL ALCOHOL/SMDI COPOLYMER) par la Société Rohm & Haas, qui sont des polyuréthannes obtenus par condensation d'hexaméthylène diisocyanate et de polyéthylèneglycol, portant à leurs extrémités respectivement un résidu méthyle et un résidu octadécyle. Ces polyuréthannes contiennent, en plus, de 3 à 5 % en poids d'une matrice d'amidon modifié par voie enzymatique. On peut citer aussi les produits commercialisés sous les dénominations RHEOLATE® 205, 210, 212, 216, 244, 278, 255, 266, 288, 300, 350 par la Société Elementis ou encore les produits commercialisés sous les dénominations BORCHIGEL LW.44, L.75.N ; L 76 ; VP 9628-LL36 ; VP 97105-NT40 ; VP 9620 par la Société Borchers.

Parmi les composés oxyéthylénés cités ci-dessus, les composés préférés sont notamment les esters d'acides gras et de polyéthylène glycol et/ou de polypropylène glycol, et en particulier le distéarate de polyéthylène glycol (150 OE), le distéarate de polyéthylène glycol (250 OE), le PEG-150 dibehenate et le palmito-stéarate de polyéthylène glycol (120 OE) ; les dérivés alkyl ou acyl éthoxylés de polyol et en particulier le di-oléate de méthyl-glucose oxyéthyléné (120 OE) et le tétra-stéarate de pentaérythrityle oxyéthyléné (150 OE); les dérivés uréthane éthoxyéthylénés modifiés par des chaînes alkyle ; et leurs mélanges.

### Tensioactifs moussants

La composition de nettoyage selon l'invention constitue une composition moussante, et elle contient au moins un tensioactif moussant qui va apporter le caractère moussant à la composition. Ce tensioactif peut être choisi parmi tous les tensioactifs moussants non ioniques, anioniques, amphotères et zwitterioniques, et leurs mélanges.

La quantité de tensioactif(s) va en poids de matière active, de 3 à 50 % en poids et mieux de 3 à 30 % en poids par rapport au poids total de la composition.

### 1. Les tensioactifs non ioniques :

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl N-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Henkel, et sous la dénomination ORAMIX NS 10® par la société Seppic ; le caprylyl/capryl glucoside comme le produit commercialisé sous la dénomination ORAMIX CG 110 ® par la Société Seppic ; le laurylglucoside comme les produits commercialisés sous les dénominations PLANTAREN 1200 N® et PLANTACARE 1200® par la société Henkel ; et le coco-glucoside comme le produit commercialisé sous la dénomination PLANTACARE 818/UP® par la société Henkel.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

### 2. Les tensioactifs anioniques

On peut utiliser par exemple comme tensioactifs anioniques, les carboxylates, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkyl polyglucoside, les savons d'acides gras et leurs mélanges. Si les savons d'acides gras peuvent être ajoutés à la composition de l'invention, leur quantité doit être telle que leur addition ne porte pas préjudice aux qualités cosmétiques de la composition obtenue.

Comme carboxylates, on peut citer par exemple les sels alcalins de N-acylaminoacides ; les amido éthercarboxylates (AEC) comme le lauryl amido ether carboxylate de sodium (3 OE) commercialisé sous la dénomination AKYPO FOAM 30® par la société Kao Chemicals ; les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxyméthyle, comme le produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX ® par la société Nikkol.

Les dérivés des aminoacides peuvent être choisis par exemple parmi les sarcosinates et notamment les acylsarcosinates comme le lauroyl sarcosinate de sodium commercialisé sous la dénomination SARKOSYL NL 97® par la société Ciba ou sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; les alaninates comme le N-lauroyl-N-methylamidopropionate de sodium, commercialisé sous la dénomination SODIUM NIKKOL ALANINATE LN 30® par la société Nikkol ou sous la dénomination ALANONE ALE® par la société Kawaken, et le N-lauroyl N-methylalanine triéthanolamine, commercialisé sous la dénomination ALANONE ALTA® par la société Kawaken ; les N-acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; les aspartates comme le mélange de N-lauroylaspartate de triéthanolamine et de N-myristoylaspartate de triéthanolamine, commercialisé sous la dénomination ASPARACK® par la société Mitsubishi ; les citrates, et leurs mélanges.

Comme alkyl éther sulfates, on peut citer par exemple le lauryl éther sulfate de sodium (C12-14 70/30) (2,2 OE) commercialisé sous les dénominations SIPON AOS 225® ou TEXAPON N702 PATE® par la société Henkel, le lauryl éther sulfate d'ammonium (C12-14 70/30) (3 OE) commercialisé sous la dénomination SIPON LEA 370® par la société Henkel, l'alkyl (C12-C14) éther (9 OE) sulfate d'ammonium commercialisé sous la dénomination RHODAPEX AB/20® par la société Rhodia Chimie.

Comme sulfonates, on peut citer par exemple les alpha-oléfines sulfonates comme l'alpha-oléfine sulfonate de sodium (C14-16) commercialisé sous les dénominations BIO-TERGE AS-40® et BIO-TERGE AS-40 CG® par la société Stepan, sous les dénominations WITCONATE AOS PROTEGE® et SULFRAMINE AOS PH 12® par la société Witco, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40® et MANROSOL SXS93® par la société Manro.

Comme iséthionates, on peut citer les acyliséthionates comme le cocoyl-iséthionate de sodium, tel que le produit commercialisé sous la dénomination JORDAPON CI P® par la société Jordan.

Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Henkel, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco.

Comme phosphates et alkylphosphates, on peut citer par exemple les monoalkylphosphates et les dialkylphosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR® par la société Croda ; le sel de triéthanolamine de cocoyl protéine de soja hydrolysée, commercialisé sous la dénomination MAY-TEIN SY® par la société Maybrook ; le sel de sodium des lauroyl amino-acides d'avoine, commercialisé sous la dénomination PROTEOL OAT® par la société Seppic ; l'hydrolysat de collagène greffé sur l'acide gras de coprah, commercialisé sous la dénomination GELIDERM 3000® par la société Deutsche Gelatine; les protéines de soja acylées par des acides de coprah hydrogénés, commercialisées sous la dénomination PROTEOL VS 22® par la société Seppic.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia; le sel di-sodique d'ester sulfosuccinique de cocoylpolyglucoside (1,4), commercialisé sous la dénomination ESSAI 512 MP® par la société Seppic ; le sel de sodium d'ester citrique de cocoyl polyglucoside (1,4) commercialisé sous la dénomination EUCAROL AGE-EC® par la société Cesalpinia.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les hydroxydes de métaux alcalins ou alcalino-terreux, les aminoacides, et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique ou stéarique (laurate, myristate, palmitate et stéarate de potassium ou de sodium), et leurs mélanges.

### 3. Les tensioactifs amphotères et zwitterioniques

On peut utiliser par exemple comme tensioactifs amphotères et zwitterioniques, les bétaïnes, les N-alkylamidobétaïnes et leurs dérivés, les dérivés de la glycine, les sultaïnes, les alkyl-polyaminocarboxylates, les alkylamphoacétates et leurs mélanges.

Comme bétaïnes, on peut citer par exemple la cocobétaïne comme le produit commercialisé sous la dénomination DEHYTON AB-30® par la société Henkel ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme dérivés de la glycine, on peut citer le N-cocoylglycinate de sodium commercialisé sous la dénomination AMILITE GCS-12® par la société Ajinomoto.

Comme sultaines, on peut citer le cocoyl-amidopropylhydroxy-sulfobetaine commercialisé sous la dénomination CROSULTAINE C-50® par la société Croda.

Comme alkyl-polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel; le stéaryl-polyamidocarboxylate de sodium commercialisé sous la dénomination AMPHOLAK 7 TX/C® par la société Akzo Nobel; la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacetate).

Parmi les tensioactifs cités ci-dessus, selon un mode particulier de réalisation de l'invention, on utilise plus particulièrement comme tensioactifs anioniques, les acylsarcosinates, les alkyl éther sulfates oxyéthylénés, les N-acyl N-méthyltaurates, les N-acylglutamates, les acyliséthionates, les sulfosuccinates, les phosphates et alkylphosphates, les polypeptides ; comme tensioactifs amphotères et zwitterioniques, les bétaïnes et les alkylamphoacétates ; comme tensioactifs non ioniques, les alkylpolyglucosides, l'O-octanoyl-6'-D-maltose, l'O-dodecanoyl-6'-D-maltose, le dodecanediol polyglycérolé (3,5 moles de glycérol), l'éthyl-2 hexyl oxy-carbonyl N-methyl glucamine ; et les mélanges de ces tensioactifs.

Selon un mode encore plus particulièrement préféré de réalisation de l'invention, les tensioactifs sont choisis parmi les phosphates et alkylphosphates, les alkylpolyglucosides, et leurs mélanges, avec adjonction éventuelle des autres tensioactifs cités, et en particulier parmi le decylglucoside, le caprylyl/capryl glucoside, le laurylglucoside, le coco-glucoside, le mono-phosphate de lauryle, le sel de potassium de l'acide dodécyl-phosphorique, le mélange de monoester et de di-ester d'acide octylphosphorique, le sel de potassium ou de triéthanolamine de mono-alkyl (C12-C13) phosphate, le lauryl phosphate de potassium, et leurs mélanges.

### Polymères

La quantité de polymère(s) cationique(s) et/ou amphotère(s) dans la composition de l'invention peut aller par exemple de 0,01 à 5 % en poids de matière active et de préférence de 0,1 à 2 % en poids de matière active par rapport au poids total de la composition.

On peut utiliser un ou plusieurs polymères cationiques et/ou amphotères. Selon un mode préféré de réalisation de l'invention, la composition contient au moins un polymère cationique.

### Polymères cationiques

On peut citer par exemple comme polymères cationiques utilisables dans la composition de l'invention, les polymères comportant au moins un groupement amine quaternaire et éventuellement des groupements amine primaire, secondaire, tertiaire et/ou quaternaire faisant partie de la chaîne polymère ou directement reliés à celle-ci, les dits polymères ayant un poids moléculaire allant de 500 à environ 5.000.000 et de préférence de 1000 à 3.000.000. Ils sont également caractérisés par une densité de charge allant de 0,9 à 7 meq/g et de préférence de 0,9 à 4 meq/g.

Parmi ces polymères, on peut citer plus particulièrement les polymères cationiques suivants :
(1) les homopolymères ou copolymères obtenus à partir d'un ou plusieurs monomères insaturés et comportant au moins un groupe ammonium quaternaire, et en particulier un des motifs de formules suivantes : 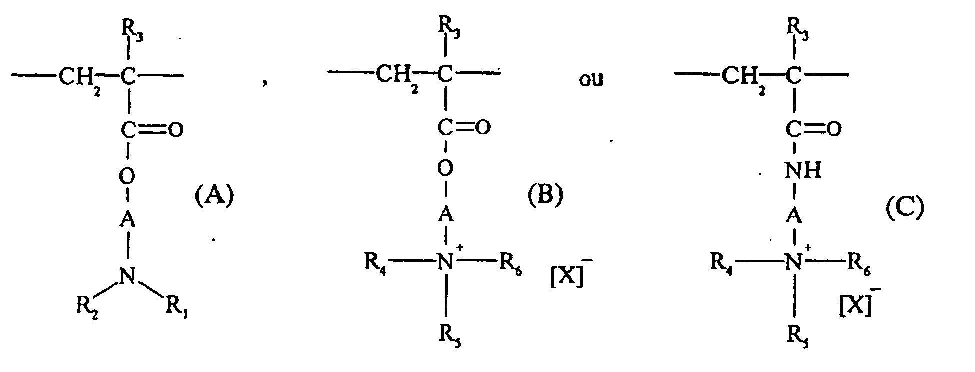 dans lesquelles :
   R₃ désigne un atome d'hydrogène ou un radical CH₃ ;
   A est un groupe alkylène linéaire ou ramifié comportant de 1 à 6 atomes de carbone ou un groupe hydroxyalkylène comportant de 1 à 4 atomes de carbone ;
   R₄, R₅ et R₆, identiques ou différents, représentent un groupe alkyle ayant de 1 à 18 atomes de carboné ou un radical benzyle ;
   R₁ et R₂ représentent un atome d'hydrogène ou un groupe alkyle ayant de 1 à 6 atomes de carbone ;
   X désigne un anion méthosulfate ou un halogénure tel que chlorure ou bromure.
   Le monomère insaturé peut être choisi dans le groupe comprenant les acrylamides, méthacrylamides, diacétones acrylamides ainsi que les acrylamides et méthacrylamides substitués sur l'azote par des radicaux alkyle comportant de 1 à 8 atomes de carbone, les acides acryliques ou méthacryliques ou leurs esters, les vinyllactames tels que la vinylpyrrolidone ou le vinylcaprolactame, les esters vinyliques, et leurs mélanges.
   Ainsi, parmi ces copolymères de la famille (1), on peut citer par exemple :
   - les copolymères d'acrylamide et de diméthylaminoéthyl méthacrylate quaternisé avec du sulfate de diméthyle ou avec un halogénure de diméthyle, tels que le produit commercialisé sous la dénomination HERCOFLOC® par la société Hercules ;
   - les copolymères d'acrylamide et de chlorure de méthacryloyl-oxyéthyl-triméthylammonium, comme le POLYQUATERNIUM 15 (nom CTFA) commercialisé par exemple sous la dénomination ROHAGIT KF 720 F® par la société Röhm & Haas ;
   - le copolymère d'acrylamide et de méthosulfate de méthacryloyl-oxyéthyl-triméthylammonium comme le POLYQUATERNIUM 5 (nom CTFA) commercialisé par exemple sous la dénomination MERQUAT 5® par la société Calgon ;
   - les copolymères vinylpyrrolidone / acrylate ou méthacrylate de dialkylamino-alkyle quaternisés, comme le POLYQUATERNIUM 11 (nom CTFA) commercialisé par exemple sous les dénominations GAFQUAT 755®, GAFQUAT 755N® et GAFQUAT 734® par la société ISP ;
   - les terpolymères méthacrylate de diméthylaminoéthyle / vinylcaprolactame / vinylpyrrolidone, tels que le produit vendu sous la dénomination GAFFIX VC® 713 par la société ISP ;
   - les terpolymères méthacrylate de diméthylaminopropyle / methacrylamide / vinylpyrrolidone comme le POLYQUATERNIUM 28 (nom CTFA) commercialisé par exemple sous la dénomination GAFQUAT HS-100® par la société ISP ;
   - les copolymères à base de vinylpyrrolidone et vinylcaprolactame comme le POLYQUATERNIUM 46 (nom CTFA) commercialisé par exemple sous la dénomination LUVIQUAT HOLD® par la société BASF ;
(2) les homopolymères ou les copolymères obtenus à partir d'un ou plusieurs monomères insaturés et comportant un radical dimethyldiallylammonium de préférence de formule (IV) décrite ci dessous 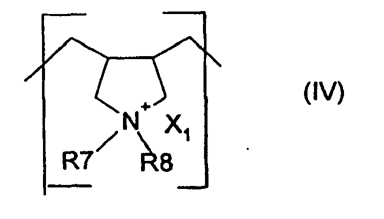 dans laquelle :
   R₇ et R₈, identiques ou différents, désignent un atome d'hydrogène ou représentent un groupe alkyle ayant de 1 à 18 atomes de carbone ;
   X₁ désigne un anion méthosulfate ou un halogénure tel que chlorure ou bromure.
   Le monomère insaturé peut être choisi dans le groupe comprenant les acrylamides, les méthacrylamides, les diacétones acrylamides, les acrylamides et méthacrylamides substitués sur l'azote par des radicaux alkyle comportant de 1 à 8 atomes de carbone, les acides acryliques ou méthacryliques ou leurs esters, les vinyllactames tels que la vinylpyrrolidone ou le vinylcaprolactame, les esters vinyliques, et leurs mélanges.
   Ainsi, parmi les copolymères de la famille (2), on peut citer par exemple:
   - les polymères de chlorure de dimethyldiallyl ammonium, comme le POLYQUATERNIUM 6 (nom CTFA) commercialisé par exemple sous les dénominations SALCARE SC 30® par la société Ciba, et MERQUAT 100® par la société Calgon ;
   - les copolymères d'acrylamide et de chlorure de diméthyldiallyl ammonium, comme le POLYQUATERNIUM 7 (nom CTFA) commercialisé par exemple sous les dénominations MERQUAT S®, MERQUAT 2200® et MERQUAT 550® par la société Calgon et SALCARE SC 10® par la société Ciba ;
(3) les copolymères quaternaires de vinylpyrrolidone et de vinylimidazole ; comme le POLYQUATERNIUM 16 (nom CTFA) commercialisé par exemple sous les dénominations LUVIQUAT FC905®, LUVIQUAT FC370®, LUVIQUAT HM552® et LUVIQUAT FC550® par la société BASF, et le POLYQUATERNIUM 44 (nom CTFA) commercialisé par exemple sous la dénomination LUVIQUAT CARE® par la société BASF ;
(4) les polysaccharides quaternisés tels que :
   - les gommes de guar ou d'hydroxypropyl guar contenant des groupements cationiques trialkylammonium, comme les produits commercialisés notamment sous les dénominations commerciales de JAGUAR C13 S®, JAGUAR C 15®, JAGUAR C 17®, JAGUAR C 162®, JAGUAR C 2000®, JAGUAR EXCEL® par la société Meyhall ;
   - les dérivés de cellulose quaternisés comme les polymères d'hydroxyéthylcellulose contenant des groupes cationiques trialkylammonium et notamment triméthylammonium, comme le POLYQUATERNIUM 10 (nom CTFA) commercialisé par exemple sous les dénominations UCARE POLYMER JR-400®, UCARE POLYMER JR-125®, UCARE POLYMER LR-400® par la société Amerchol ; et comme les dérivés de cellulose greffés avec un monomère hydrosoluble comportant un ammonium quaternaire, notamment ceux décrits dans le brevet US-4,131,576, tels que les hydroxyalkylcelluloses greffées, par exemple les hydroxyméthyl-, hydroxyéthyl- ou hydroxypropylcelluloses greffées avec un sel de méthacryloyloxyéthyl-triméthylammonium, de méthacrylamidopropyl-triméthyl-ammonium, ou de diméthyl-diallylammonium, plus particulièrement le copolymère d'hydroxyéthyl-cellulose et de chlorure de diméthyldiallylammonium (nom CTFA: POLYQUATERNIUM 4) commercialisé sous les dénominations CELQUAT L 200® et CELQUAT H 100® par la Société National Starch.
(5) les chitosanes ou leurs sels tels que les acétate, lactate, glutamate, gluconate ou le pyrrolidone carboxylate de chitosane. Parmi ces composés, on peut citer notamment le chitosane ayant un taux de désacétylation de 90,5% en poids vendu sous la dénomination KYTAN BRUT STANDARD® par la société Aber Technologies, et le pyrrolidone carboxylate de chitosane vendu sous la dénomination KYTAMER PC® par la société Amerchol.
et (6) les mélanges de ces polymères cationiques.

Les polymères cationiques particulièrement préférés sont les POLYQUATERNIUM 5, POLYQUATERNIUM 7, POLYQUATERNIUM 28, POLYQUATERNIUM 39, POLYQUATERNIUM 44, et leurs mélanges, car ils apportent beaucoup de douceur au produit fini, tout en conduisant, en présence de silice, à un rinçage optimum.

### Polymères amphotères

Comme polymères amphotères utilisables dans la composition, on peut citer les polymères comportant des motifs K et M répartis statistiquement dans la chaîne polymère, dans lesquels K et M peuvent avoir les significations suivantes :
1) K désigne un motif dérivant d'un monomère comportant au moins un atome d'azote basique et M désigne un motif dérivant d'un monomère acide comportant un ou plusieurs groupements carboxyliques ou sulfoniques, ou bien
2) K et M désignent des groupements dérivant de monomères zwitterioniques de carboxybétaïnes ou de sulfobétaïnes; ou bien
3) K et M désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ; ou bien
4) K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkyl-méthacrylates et acrylates, les dialkylaminoalkyl-méthacrylamides et acrylamides. On peut citer par exemple le copolymère acrylate de sodium / chlorure d'acrylamidopropyl-trimethylammonium vendu sous la dénomination POLYQUART KE 3033 par la Société HENKEL ; les terpolymères acide acrylique / acrylate de methyle / chlorure de méthacrylamido-propyltrimonium, comme le POLYQUATERNIUM 47 (nom CTFA) commercialisé par exemple sous la dénomination MERQUAT 2001 N® par la société Calgon.
   Le composé vinylique peut être également un sel de dialkyldiallylammonium tel que le chlorure de diéthyldiallylammonium. Les copolymères d'acide acrylique et de chlorure de diéthyldiallylammonium sont par exemple ceux commercialisés sous les appellations MERQUAT 280®, MERQUAT 295® et MERQUAT PLUS 3330® par la société Calgon.
(2) Les polymères comportant des motifs dérivant :
   a) d'au moins un monomère choisi parmi les acrylamides ou les méthacrylamides substitués sur l'azote par un radical alkyle, notamment les acrylamides ou méthacrylamides N-alkyl-substitués dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants ;
   b) d'au moins un comonomère acide contenant un ou plusieurs groupements carboxyliques réactifs, tels que les acides acrylique, méthacrylique, crotonique, itaconique, maléique, fumarique ainsi que les monoesters d'alkyle ayant 1 à 4 atomes de carbone des acides ou des anhydrides maléique ou fumarique ; et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle. Les comonomères basiques préférés sont des méthacrylates d'aminoéthyle, de butyl aminoéthyle, de N,N-diméthylaminoéthyle, de N-tertio-butylaminoéthyle.
   Comme polymères comportant ces motifs, on peut citer le produit vendu sous les dénominations AMPHOMER® ou LOVOCRYL 47® par la société National Starch (nom CTFA: Octylacrylamide / acrylates / butylaminoethylmethacrylate copolymer).
(3) Les polyaminoamides réticulés et alcoylés (partiellement ou totalement), qui sont réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
(4) Les polymères comportant des motifs zwitterioniques de formule (V) : 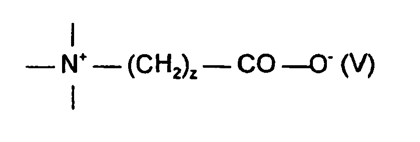 Les polymères comprenant de telles unités peuvent également comporter des motifs dérivés de monomères non zwitterioniques tels que l'acrylate ou le méthacrylate de diméthyl- ou diéthyl-aminoéthyle ou des alkyl-acrylates ou alkyl-méthacrylates, des acrylamides ou méthacrylamides ou l'acétate de vinyle.
   A titre d'exemple, on peut citer le copolymère de méthacryloylethylN,N-diméthyl-carboxyméthylbétaïne/méthacrylate de butyle tel que le produit vendu sous la dénomination DIAFORMER Z301® par la société Sandoz.
(5) les polymères amphotères dérivés du chitosane comme les polymères dérivés de la N-carboxyalkylation du chitosane tel que le N-carboxyméthyl chitosane ou le N-carboxybutyl chitosane vendu sous la dénomination EVALSAN® par la société Jan Dekker.
(6) Les polymères amphotères dérivés de l'acide chloracétique ou du chloracétate de sodium.
(7) Les copolymères alkyl(C₁-C₅)-vinyléther / anhydride maléique modifié partiellement par semi-amidification avec une N,N-dialkylaminoalkylamine telle que la N,N-diméthylaminopropylamine ou par semi-estérification avec une N,N-dialcanolamine. Ces copolymères peuvent également comporter d'autres comonomères vinyliques tels que le vinylcaprolactame.

On peut aussi utiliser un mélange de ces polymères amphotères, ou un mélange de polymères cationiques et amphotères.

Les polymères amphotères particulièrement préférés selon l'invention sont par exemple les produits commercialisés sous les dénominations MERQUAT 2001®, MERQUAT 280®, MERQUAT 295® et MERQUAT PLUS 3330® par la société Calgon.

Le milieu aqueux de la composition selon l'invention peut comprendre, outre l'eau, un ou plusieurs solvants choisis parmi les alcools inférieurs comportant de 1 à 6 atomes de carbone, tels que l'éthanol ; les polyols, tels que par exemple la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller par exemple de 0,5 à 30 % en poids et de préférence de 2 à 20 % en poids par rapport au poids total de la composition.

Les compositions de l'invention peuvent comprendre des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les nacres, les charges minérales ou organiques, matifiantes, blanchissantes ou exfoliantes, les colorants solubles, les actifs cosmétiques ou dermatologiques, les polymères non ioniques tels que le polyvinylpyrrolidone (PVP), les polymères anioniques, les corps gras incompatibles avec le milieu aqueux, comme les huiles ou les cires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme actifs, on peut utiliser dans la composition de l'invention tout actif habituellement utilisé dans les domaines cosmétique et dermatologique, tels que par exemple les vitamines ou provitamines hydrosolubles ou liposolubles comme les vitamines A (rétinol), C (acide ascorbique), B3 ou PP (niacinamide), B5 (panthénol), E (tocophérol), K1, le bêta-carotène, et les dérivés de ces vitamines et notamment leurs esters ; les stéroïdes comme la DHEA et la 7α-hydroxy DHEA ; les antiseptiques ; les antisébohrréïques et antimicrobiens tels que le peroxyde de benzoyle, l'acide salicylique, le triclosan, le triclocarban, l'acide azélaïque ; les hydratants comme la glycérine, l'acide hyaluronique, le pyrrolidone carboxylique acide (PCA) et ses sels, le pidolate de sodium, la sérine, le xylitol, le tréhalose, l'ectoïne, les céramides, l'urée ; les agents kératolytiques et anti-âge tels que les alpha-hydroxyacides comme l'acide glycolique, l'acide citrique, l'acide lactique, les béta-hydroxyacides comme l'acide salicylique et ses dérivés ; les enzymes et co-enzymes et notamment le coenzyme Q10 ; les filtres solaires ; les azurants optiques ; les actifs amincissants comme la caféine, la théophyline, la théobromine, les anti-inflammatoires tels que les acides 18 β glycyrrhétinique et ursolique, et leurs mélanges. On peut utiliser un mélange de deux ou plusieurs de ces actifs. Le ou les actifs peuvent être par exemple présents en une concentration allant de 0,01 à 20 %, de préférence de 0,1 à 10 % et mieux de 0,5 à 5 % du poids total de la composition.

Comme charges, on peut citer les charges minérales telles que le talc ou silicate de magnésium (granulométrie: 5 microns) commercialisé sous la dénomination LUZENAC 15 M00® par la société Luzenac, le kaolin ou silicate d'aluminium comme par exemple celui commercialisé sous la dénomination KAOLIN SUPREME® par la société Imerys, ou les charges organiques telles que l'amidon comme par exemple le produit commercialisé sous la dénomination AMIDON DE MAIS B ® par la société Roquette, les microsphères de Nylon comme celles commercialisées sous la dénomination ORGASOL 2002 UD NAT COS® par la société Atochem, les microsphères à base de copolymère de chlorure de vinylidene/Acrylonitrile/methacrylonitrile enfermant de l'isobutane, expansées comme celles commercialisées sous la dénomination EXPANCEL 551 DE® par la société Expancel. On peut ajouter aussi à la composition de l'invention, des fibres comme par exemple des fibres de nylon (POLYAMIDE 0.9 DTEX 0.3 MM commercialisé par les Etablissements Paul Bonte), des fibres de cellulose ou « Rayonne » (RAYON FLOCK RCISE NOOO3 MO4® commercialisé par la société Claremont Flock Corporation).

Les compositions moussantes selon l'invention peuvent être utilisées dans les domaines cosmétique et dermatologique, et elles peuvent constituer notamment des produits de nettoyage ou de démaquillage de la peau (corps, visage, yeux), du cuir chevelu et/ou des cheveux. Elles peuvent être utilisées pour tout type de peau (sèche, normale, mixte ou grasse).

Un autre objet de l'invention consiste en l'utilisation cosmétique de la composition telle que définie ci-dessus, comme produits de nettoyage et/ou de démaquillage de la peau, des yeux, du cuir chevelu et/ou des cheveux.

Les compositions selon l'invention peuvent constituer aussi une composition pour désinfecter la peau et/ou le cuir chevelu, notamment quand elles contiennent un antibactérien. On peut aussi les utiliser pour le traitement des peaux grasses, notamment en y ajoutant des actifs spécifiques de traitement des peaux grasses, tels que les antiséborrhéiques comme par exemple l'acide salicylique et ses dérivés, l'acide azélaïque, le triclosan, le triclocarban, le piroctone olamine, la niacinamide (vitamine PP).

Un autre objet de l'invention est l'utilisation de la composition telle que définie ci-dessus pour la préparation d'une composition destinée à désinfecter la peau et/ou le cuir chevelu.

Un autre objet de l'invention consiste en un procédé cosmétique de nettoyage de la peau, des yeux, du cuir chevelu et/ou des cheveux, caractérisé par le fait qu'on applique la composition de l'invention, sur la peau, sur les yeux, sur le cuir chevelu et/ou sur les cheveux, en présence d'eau, et qu'on élimine la mousse formée par rinçage à l'eau.

Dans le cas du nettoyage du visage, la composition selon l'invention peut constituer un masque qui est rincé après un temps de pose de 1 à 3 minutes.

Les exemples qui suivent servent à illustrer l'invention sans toutefois présenter un caractère limitatif. Les quantités indiquées sont en % en poids sauf mention contraire. L'indication « M.A. » signifie « matière active ». Ces exemples sont des compositions sans savon.

### Exemples 1 à 4 et exemples comparatifs 1 et 2 :

Le tableau (4) présente les exemples 1 à 4 et les exemples comparatifs 1 et 2.

L'exemple comparatif 1 correspond à l'exemple 1 ou l'exemple 2 selon l'invention sans polymère cationique, et l'exemple comparatif 2 correspond à l'exemple 2 selon l'invention sans silice.

Les exemples comparatifs sont notés « ex. comp. » dans les tableaux ci-dessous.

Les exemples 1 à 4 selon l'invention diffèrent entre eux par le polymère cationique utilisé.

**Tableau (4)**

| Composition | Ex. 1 | Ex. comp. 1 | Ex. 2 | Ex. comp. 2 | Ex. 3 | Ex. 4 |
|---|---|---|---|---|---|---|
| Mono-phosphate de lauryle (Mono-ester : 75 %) (MAP 20 de KAO) | 3,35 M.A. | 3,35 M.A. | 3,35 M.A. | 3,35 M.A. | 3,35 M.A. | 3,35 M.A. |
| Decylglucoside (MYDOL 10 de KAO à 40% M.A.) | 10,5 M.A. | 10,5 M.A. | 10,5 M.A. | 10,5 M.A. | 10,5 M.A. | 10,5 M.A. |
| Hydroxyde de potassium qs pH 7 | 0,9 | 0,9 | 0,9 | 0,9 | 0,9 | 0,9 |
| PEG-120 methyl glucose dioleate, (Glucamate DOE-120 VEGETAL d'AMERCHOL) | 2 | 2 | 2 | 2 | 2 | 2 |
| AEROSIL R-200 de DEGUSSA (hydrophile) | 5 | 5 | 5 | 0 | 5 | 5 |
| *MERQUAT 2001 N (Polyquaternium 47) | 0,5 M.A. | 0 | 0 | 0 | 0 | 0 |
| *UCARE POLYMER JR-400 (Polyquaternium 10) | 0 | 0 | 0,5 M.A. | 0,5 M.A. | 0 | 0 |
| *MERQUAT 100 (Polyquaternium 6) | 0 | 0 | 0 | 0 | 0,5 M.A. | 0 |
| *GAFQUAT 755 N (Polyquaternium 11) | 0 | 0 | 0 | 0 | 0 | 0,5 M.A. |
| Conservateurs | qs | qs | qs | qs | qs | qs |
| Eau déminéralisée | Qsp 100 % | Qsp 100 % | Qsp 100 % | Qsp 100 % | Qsp 100 % | Qsp 100 % |
| Aspect | Gel souple translucide homogène | Gel souple homogène | Gel épais légèrement translucide | Gel transparent, lisse, homogène. | Gel opaque, souple, homogène et lisse | Gel légèrement translucide |
| pH | 7 | 6,8 | 7 | 7 | 6,9 | 7 |
| Viscosité à 25°C Rhéomat 180 | 92 P. (M 4) | 111 P. (M 4) | 184 P. (M 4) | 43 P. (M 4) | 45 P. (M 4) | 54 P. (M 4) |

| | | | | | | |
|---|---|---|---|---|---|---|
| Dans le tableau (4) ci-dessus, P signifie poises et M indique le mobile de mesure de la viscosité (M 4 = mobile 4). *MERQUAT 2001 N: Terpolymère acide acrylique / chlorure de methacryl-amidopropyltrimonium / acrylate de methyle en solution aqueuse à 20% commercialisé par CALGON. Nom CTFA : Polyquaternium 47. | | | | | | |
| *UCARE POLYMER JR-400 : Hydroxyéthylcellulose quaternisée par le chlorure de 2,3-epoxypropyl trimethylammonium, commercialisée par AMERCHOL. Nom CTFA : Polyquaternium 10. | | | | | | |
| *MERQUAT 100 : Chlorure de poly-dimethyl-diallyl-ammonium dans l'eau à 40 %, commercialisé par CALGON. Nom CTFA : Polyquaternium 6. | | | | | | |
| *GAFQUAT 755 N: Copolymère vinylpyrrolidone/methacrylate de di-methyl aminoéthyle quaternisé par le sulfate de diméthyle, dans l'eau à 20 %, commercialisé par ISP. Nom INCI : Polyquaternium 11. | | | | | | |

Performances sensorielles : les qualités de mousse développées par les compositions décrites ci-dessus ont été évaluées selon le protocole décrit ci-dessous.

Avant toute utilisation des produits, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau ,
6- les essuyer.

Les critères sont évalués à chaque étape du protocole suivi, et ils sont notés sur une échelle de 0 à 10. On considère que, pour un critère donné, il y a une différence de note entre deux compositions lorsque cette différence est supérieure ou égale à 1.
- étape 3 :
   - évaluation du glissant à l'étalement : la note attribuée est d'autant plus élevée que la sensation de glissant sur les mains humides est grande.
   - évaluation du mélange à l'eau : la note attribuée est d'autant plus élevée que le mélange du produit avec l'eau est facile.
   - évaluation de l'homogénéité : la note attribuée est d'autant plus élevée que le film formé par le produit sur la main est homogène.
   - évaluation du pouvoir couvrant : la note attribuée est d'autant plus élevée que la peau de la main ne se voit pas à travers le produit étalé sur celle-ci.
- étape 4 : évaluation de la qualité de mousse
   - *Le volume de mousse:* la note attribuée est d'autant plus élevée que le volume est grand, et donc le produit est meilleur quand la note est élevée.
   - *La taille des bulles composant la mousse :* la note attribuée est d'autant plus élevée que les bulles sont grosses, et donc le produit est meilleur quand la note est basse.
   - *La densité :* consistance, tenue de la mousse ; la note attribuée est d'autant plus élevée que la densité est grande, et donc le produit est meilleur quand la note est élevée.
   - *La douceur de la mousse :* la note attribuée est d'autant plus élevée que la mousse est douce, et donc le produit est meilleur quand la note est élevée.
- étape 5 : évaluation pendant le rinçage
   - *Le rinçage :* la note attribuée est d'autant plus faible que la présence d'un film glissant difficile à éliminer est grande, et donc le produit est meilleur quand la note est élevée.

Les résultats sensoriels pour chacun des critères sont présentés dans le tableau (5) suivant :

**Tableau (5)**

| | Ex. 1 | Ex. comp.1 | Ex. 2 | Ex. Comp.2 | Ex. 3 | Ex. 4 |
|---|---|---|---|---|---|---|
| Glissant à l'étalement | 7,8 | 7,7 | 7,5 | 7,8 | 7,2 | 8,2 |
| Mélange à l'eau | 7,5 | 7,2 | 6,3 | 7,0 | 7,5 | 6,2 |
| Homogénéité | 5,7 | 7,0 | 5,5 | 5,8 | 7,5 | 6,0 |
| Pouvoir couvrant | 6,5 | 7,0 | 6,3 | 7,5 | 7,0 | 7,0 |
| Volume de la mousse | 4,3 | 5,3 | 4,8 | 5,0 | 5,8 | 5,3 |
| Taille des bulles | 4,0 | 4,7 | 3,8 | 3,5 | 5,5 | 3,1 |
| Densité | 7,0 | 7,5 | 7,5 | 9,0 | 6,7 | 7,3 |
| Douceur de la mousse | 7,8 | 7,0 | 7,1 | 8,5 | 6,7 | 7,5 |
| Rinçage | 9,7 | 9,7 | 8,3 | 6,3 | 9,7 | 10 |

Les exemples décrits ci-dessus démontrent l'influence positive de la silice sur le rinçage des compositions à base de polymères cationiques. Ainsi, malgré la présence des polymères Polyquaternium 47, Polyquaternium 6 et Polyquaternium 11, les exemples 1, 3 et 4 selon l'invention ont un rinçage très bon (note de 9,7 ou 10), équivalent à celui de l'exemple comparatif 1 qui ne contient pas de polymère, bien que ces polymères soient connus comme affectant la qualité du rinçage.

Dans le cas du Polyquaternium 10 connu pour son fini filmogène, l'exemple 2 montre que la silice permet d'améliorer notablement le rinçage par rapport à l'exemple comparatif 2 qui ne contient pas de silice, puisque la note de rinçage est alors de 8,3 au lieu de 6,3, tout en conservant une douceur satisfaisante.

Exemples 5 et 6 et exemples comparatifs 3 à 9: composition à base de Polyquaternium 7 (copolymère d'acrylamide et de chlorure de diméthyl di-allylammonium). Ces exemples sont présentés dans le tableau (6).
Les exemples 5 et 6 diffèrent l'un de l'autre par le type de silice utilisée et par la présence de glycérine dans l'exemple 6.

Les exemples comparatifs 3 et 4 sont à comparer aux exemples 5 et 6, les exemples comparatifs 5 à 7 sont à comparer à l'exemple 5, et les exemples comparatifs 8 et 9 sont à comparer à l'exemple 6 :
- Ex. comparatif 3 : correspond à l'exemple 5 ou 6 sans silice ni polymère cationique.
- Ex. comparatif 4 : correspond à l'exemple 5 ou 6 sans silice.
- Ex. comparatif 5 : correspond à l'exemple 5 sans polymère cationique ni composé oxyéthyléné.
- Ex. comparatif 6 : correspond à l'exemple 5 sans polymère cationique.
- Ex. comparatif 7 : correspond à l'exemple 5 sans composé oxyéthyléné.
- Ex. comparatif 8: correspond à l'exemple 6 sans polymère cationique ni composé oxyéthyléné.
- Ex. comparatif 9 : correspond à l'exemple 6 sans polymère cationique.

**Tableau (6)**

| Compositions | Ex. comp 3 | Ex. comp 4 | Ex. comp 5 | Ex. comp 6 | Ex. comp 7 | Ex. 5 | Ex. comp 8 | Ex. comp 9 | Ex. 6 |
|---|---|---|---|---|---|---|---|---|---|
| Decylglucoside (MYDOL 10 de Kao à 40% de M.A.) | 6,5 % M.A. | 6,5 % M.A. | 6,5 % M.A. | 6,5 % M.A. | 6,5 % M.A. | 6,5% M.A. | 6,5 % M.A. | 6,5 % M.A. | 6,5 % M.A. |
| Mono-phosphate de lauryle (MAP 20 de Kao) | 6,5 % M.A. | 6,5 % M.A. | 6,5 % M.A. | 6,5 % M.A. | 6,5 % M.A. | 6,5 % M.A. | 6,5 % M.A. | 6,5 % M.A. | 6,5 % M.A. |
| PEG-120 methyl glucose dioleate (Glucamate DOE-120 VEGETAL de Amerchol) | 2 | 2 | 0 | 2 | 0 | 2 | 0 | 2 | 2 |
| Polyquaternium-7 (MERQUAT S de Calgon) | 0 | 0,5 % M.A. | 0 | 0 | 0,5 % M.A. | 0,5 % M.A. | 0 | 0 | 0,5% M.A. |
| AEROSIL R-200 (hydrophile) | 0 | 0 | 5 | 5 | 5 | 5 | 0 | 0 | 0 |
| AEROSIL R-972 (hydrophobe) | 0 | 0 | 0 | 0 | 0 | 0 | 5 | 5 | 5 |
| Glycérine | 0 | 0 | 0 | 0 | 0 | 0 | 0 | 0 | 3,5 |
| Conservateurs | qs | qs | qs | qs | qs | qs | qs | qs | qs |
| Hydroxyde de potassium qs pH 7 | 1,7 | 1,7 | 1,7 | 1,7 | 1,7 | 1,7 | 1,7 | 1,7 | 1,7 |
| eau | Qsp 100 % | Qsp 100 % | Qsp 100 % | Qsp 100 % | Qsp 100 % | Qsp 100 % | Qsp 100 % | Qsp 100 % | Qsp 100 % |
| Viscosité à T0* à 25°C Rhéomat 180 | 95 P (M 4) | 165 P (M 4) | 9,1 P (M 3) | 156 P (M 4) | Non mesurable | 161 P (M 4) | < 130 cP (M 2) | 142 P (M 4) | 104 P (M 4) |
| Stabilité | Oui | Oui | Non | Oui | Non | Oui | Non | Oui | Oui |

| | | | | | | | | | |
|---|---|---|---|---|---|---|---|---|---|
| * T0 signifie au temps zéro. Dans le tableau ci-dessus, P signifie poises, cP signifie centipoises et M indique le mobile de mesure de la viscosité (M 4 = mobile 4 ; M3 = mobile 3 et M 2 = mobile 2) | | | | | | | | | |

Les exemples ont l'aspect suivant :
- Exemple comparatif 3 : Gel assez épais translucide homogène ;
- Exemple comparatif 4 : Gel assez épais translucide homogène ;
- Exemple comparatif 5 : Pâte souple opaque blanchâtre ;
- Exemple comparatif 6 : Gel très épais légèrement translucide ;
- Exemple comparatif 7 : Gel granuleux avec relargage immédiat lors de la fabrication ;
- Exemple 5 selon l'invention : Gel très épais homogène légèrement translucide ;
- Exemple comparatif 8 : Solution opaque blanche ;
- Exemple comparatif 9 : Gel très épais homogène légèrement translucide ;
- Exemple 6 selon l'invention: Gel épais souple homogène légèrement translucide.

Le tableau ci-dessus montre que :
- L'exemple 5 présente une bonne stabilité contrairement à l'exemple comparatif 5 qui ne contient ni composé oxyéthyléné ni polymère cationique et à l'exemple comparatif 7 qui ne contient pas de composé oxyéthyléné.
- L'exemple 6 présente une bonne stabilité contrairement à l'exemple comparatif 8 qui ne contient ni composé oxyéthyléné ni polymère cationique.

Performances sensorielles: elles ont été évaluées selon le protocole décrit précédemment.

Les résultats sont indiquées dans le tableau (7) ci-dessous :

**Tableau (7)**

| | Ex. comp 3 | Ex. comp 4 | Ex. comp 5 | Ex. comp 6 | Ex. 5 | Ex. comp 8 | Ex. comp 9 | Ex.6 |
|---|---|---|---|---|---|---|---|---|
| Mélange à l'eau | 6,5 | 8,2 | 8,9 | 7,3 | 9,2 | 10 | 7,5 | 10 |
| Pouvoir couvrant | 6,8 | 7 | 6,3 | 7,1 | 8,8 | 6,1 | 6,5 | 9,4 |
| Volume de mousse | 5,6 | 6 | 5,8 | 5,5 | 6,3 | 5,5 | 6,1 | 6,8 |
| Taille des bulles | 3,9 | 4,1 | 3,6 | 3,5 | 4,1 | 4,1 | 3,8 | 3,9 |
| Densité | 7,5 | 8 | 7 | 7 | 8 | 6,8 | 7,1 | 7,9 |
| Douceur de la mousse | 6,8 | 8,2 | 5,3 | 6,6 | 8,3 | 6 | 5,8 | 8,6 |
| Rinçage | 8,9 | 8,3 | 9,1 | 9,3 | 9,5 | 9,1 | 9,6 | 9,3 |

On observe une nette amélioration du pouvoir couvrant pour les compositions selon l'invention : le pouvoir couvrant des exemples 5 et 6 est nettement meilleur que celui des exemples comparatifs. C'est également avec les compositions selon l'invention que le volume de mousse est le meilleur.

D'autre part, l'addition de silice hydrophile (exemple 5) ou hydrophobe (exemple 6) permet un meilleur rinçage que l'exemple comparatif 4 contenant le polymère cationique (MERQUAT S) sans silice.

Par ailleurs, on observe une synergie pour les produits associant silice hydrophile, polymère cationique et composé oxyéthyléné, en ce qui concerne le mélange à l'eau : en effet l'exemple 5 possède une meilleure capacité à se mélanger à l'eau que les exemples comparatifs 3, 4 et 6, ce qui rend le produit plus facile à mettre en oeuvre.

### Exemples 7 à 10 selon l'invention

Ces exemples sont présentés dans le tableau (8) ci-dessous.

Les exemples 7, 9 et 10 constituent un produit moussant pouvant être notamment utilisé pour nettoyer le visage. L'exemple 8 constitue un masque moussant.

**Tableau (8)**

| Compositions | Exemple 7 | Exemple 8 | Exemple 9 | Exemple 10 |
|---|---|---|---|---|
| Decylglucoside (MYDOL 10 de Kao à 40% M.A.) | 6,5 % M.A. | 6,5 % M.A. | 6,5 % M.A. | 6,5 % M.A. |
| Mono-phosphate de lauryle (MAP 20 de Kao) | 6,5 % M.A. | 6,5 % M.A. | 6,5 % M.A. | 6,5 % M.A. |
| SER Ad Fx 1100 | 0 | 0 | 2% | 2% |
| PEG-120 méthyl glucose dioleate (GLUCAMATE DOE-120 VEGETAL d'Amerchol) | 2 % | 2 % | 0 | 0 |
| Polyquaternium 7 (MERQUAT S de Calgon) | 0,5% M.A. | 0,5% M.A. | 0,5% M.A. | 0,5% M.A. |
| AEROSIL R-200 (hydrophile) | 5 | 5 | 5 | 0 |
| AEROSIL R-972 (hydrophobe) | 0 | 0 | 0 | 5 |
| Amidon de maîs B (1) | 0 | 10 | 0 | 0 |
| Polyamide 0.9 DTEX 0,3 MM (2) | 5 | 0 | 0 | 0 |
| Conservateurs | qs | qs | qs | qs |
| Hydroxyde de potassium qs pH 7 | 1,7 | 1,7 | 1,7 | 1,7 |
| eau | Qsp100 % | Qsp100 % | Qsp100 % | Qsp100 % |
| Aspect | Gel crème blanc lisse | Gel crème blanc et lisse | Gel très épais opaque homogène | Gel épais opaque homogène |
| Viscosité à T0 à 25°C Rhéomat 180 | 109 P (M 4) | 100 P (M 4) | >236 P (M 4) | 212 P (M 4) |

| | | | | |
|---|---|---|---|---|
| Dans le tableau (8) ci-dessus, P signifie poises et M indique le mobile de mesure de la viscosité (M 4 = mobile 4). (1) Amidon de mais B : amidon de maïs : amylopectine/amylose ou « zea mays (corn) starch » commercialisé par Roquette. | | | | |
| (2) Polyamide 0.9 dtex 0.3 MM : fibres de polyamide 0.9 dtex de 0.3 mm de long, lavées à chaud puis tamponnées, ou Nylon-66 commercialisé par les établissements Paul BONTE. | | | | |

## Revendications

1. Composition de nettoyage comprenant, dans un milieu aqueux physiologiquement acceptable, (1) au moins un tensioactif moussant, (2) au moins 1 % en poids d'au moins une silice par rapport au poids total de la composition, (3) au moins un composé oxyalkyléné choisi parmi le di-oléate de méthylglucose oxyéthyléné (120 OE), le tétra-stéarate de pentaérythrityle oxyéthyléné (150 OE), le copolymère alcool stéarylique oxyéthyléné (100 OE)/polyéthylène glycol (136 OE)/hexaméthylène diisocyanate, et leurs mélanges et (4) au moins un polymère choisi parmi les polymères cationiques, les polymères amphotères et leurs mélanges., la quantité de tensioactif(s) moussant(s) allant de 3 à 50 % en poids de matière active par rapport au poids total de la composition.

2. Composition selon la revendication 1, **caractérisée en ce qu**'elle comprend au moins 35 % en poids d'eau par rapport au poids total de la composition.

3. Composition selon la revendication 1 ou 2, **caractérisée en ce qu'**elle comprend de 35 à 95 % en poids d'eau par rapport au poids total de la composition.

4. Composition selon l'une quelconque des revendications précédentes, **caractérisée en ce qu**'elle présente une viscosité allant de 0,5 à 25 Pa.s.

5. Composition selon l'une quelconque des revendications précédentes, **caractérisée en ce que** la quantité de silice(s) va de 1 à 15 % en poids de matière active par rapport au poids total de la composition.

6. Composition selon l'une quelconque des revendications précédentes, **caractérisée en ce que** la silice est choisie parmi les silices hydrophiles, les silices hydrophobes et leurs mélanges.

7. Composition selon la revendication précédente, **caractérisée en ce que** la silice hydrophile est choisie parmi les silices d'origine pyrogénée ou d'origine précipitée et leurs mélanges.

8. Composition selon la revendication 6 ou 7, **caractérisée en ce que** la silice hydrophile est choisie parmi les silices ayant une surface spécifique de 30 à 500 m²/g, une dimension moyenne de particules en nombre allant de 3 à 50 nm et une densité tassée allant de 40 à 200 g/l.

9. Composition selon l'une quelconque des revendications 6 à 8, **caractérisée en ce que** la silice hydrophile est une silice pyrogénée.

10. Composition selon l'une quelconque des revendications 6 à 8, **caractérisée en ce que** la silice hydrophile consiste en une particule enrobée de silice hydrophile.

11. Composition selon l'une quelconque des revendications 6 à 10, **caractérisée en ce que** la silice hydrophobe est choisie parmi les silices amorphes d'origine pyrogénée.

12. Composition selon l'une quelconque des revendications 6 à 11, **caractérisée en ce que** la silice hydrophobe est choisie parmi les silices ayant une surface spécifique de 50 à 500 m²/g, une dimension moyenne de particules en nombre allant de 3 à 50 nm et une densité tassée allant de 40 à 200 g/l.

13. Composition selon l'une quelconque des revendications précédentes, **caractérisée en ce que** la quantité de composé(s) oxyalkyléné(s) va de 0,1 à 20 % en poids de matière active, par rapport au poids total de la composition.

14. Composition selon l'une quelconque des revendications précédentes, **caractérisée en ce que** le tensioactif moussant est choisi parmi les tensioactifs non ioniques, les tensioactifs anioniques, les tensioactifs amphotères et zwitterioniques et leurs mélanges.

15. Composition selon l'une quelconque des revendications précédentes, **caractérisée en ce que** la quantité de tensioactif(s) moussant(s) va de 3 à 30 % en poids de matière active par rapport au poids total de la composition.

16. Composition selon l'une quelconque des revendications précédentes, **caractérisée en ce que** le tensioactif moussant est choisi parmi les alkylpolyglucosides, les esters de maltose, les alcools gras polyglycérolés, les dérivés de glucamine, les carboxylates, les dérivés des aminoacides, les alkyl sulfates, les alkyl éther sulfates, les sulfonates, les iséthionates, les taurates, les sulfosuccinates, les alkyl sulfoacétates, les phosphates et alkylphosphates, les polypeptides, les dérivés anioniques d'alkylpolyglucoside, les savons d'acide gras, les bétaïnes, les N-alkylamidobétaïnes et leurs dérivés, les dérivés de la glycine, les sultaïnes, les alkyl polyaminocarboxylates, les alkylamphoacétates, et leurs mélanges.

17. Composition selon la revendication précédente, **caractérisée en ce que** le tensioactif moussant est choisi parmi le decylglucoside, le caprylyl/capryl glucoside, le laurylglucoside, le coco-glucoside, le mono-phosphate de lauryle, le sel de potassium de l'acide dodécyl-phosphorique, le mélange de monoester et de di-ester d'acide octylphosphorique, le sel de potassium ou de triéthanolamine de mono-alkyl (C12-C13) phosphate, le lauryl phosphate de potassium, et leurs mélanges.

18. Composition selon l'une quelconque des revendications précédentes, **caractérisée en ce que** la quantité de polymère(s) cationique(s) et/ou amphotère(s) va de 0,01 à 5 % en poids de matière active par rapport au poids total de la composition.

19. Composition selon l'une quelconque des revendications précédentes, **caractérisée en ce que** le polymère est un polymère cationique ayant un poids moléculaire allant de 500 à environ 5.000.000.

20. Composition selon l'une quelconque des revendications précédentes, **caractérisée en ce que** le polymère est un polymère cationique ayant une densité de charge allant de 0,9 à 7 meq/g.

21. Composition selon l'une quelconque des revendications précédentes, **caractérisée en ce que** le polymère est un polymère cationique choisi parmi :
(1) les homopolymères ou copolymères obtenus à partir d'un ou plusieurs monomères insaturés et comportant au moins un groupe ammonium quaternaire, ayant une des formules suivantes : 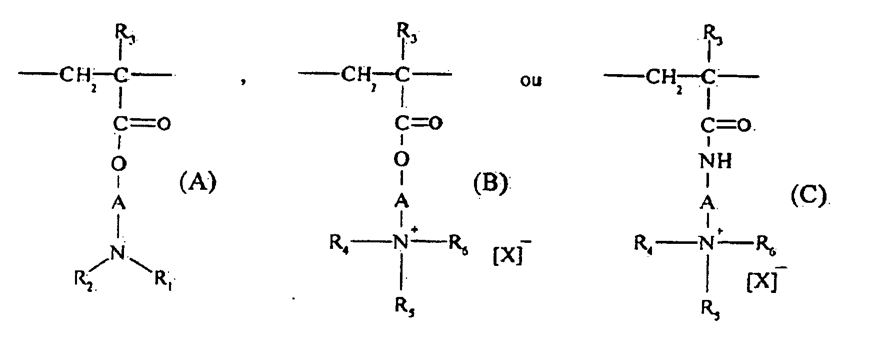 dans lesquelles :
R₃ désigne un atome d'hydrogène ou un radical CH₃;
A est un groupe alkylène linéaire ou ramifié comportant de 1 à 6 atomes de carbone ou un groupe hydroxyalkylène comportant de 1 à 4 atomes de carbone ;
R₄, R₅ et R₆, identiques ou différents, représentent un groupe alkyle ayant de 1 à 18 atomes de carbone ou un radical benzyle;
R₁ et R₂ représentent un atome d'hydrogène ou un groupe alkyle ayant de 1 à 6 atomes de carbone ;
X désigne un anion méthosulfate ou un halogénure tel que chlorure ou bromure ;
(2) les homopolymères ou copolymères obtenus à partir d'un ou plusieurs monomères insaturés et comportant un radical dimethyldiallylammonium ;
(3) les copolymères quaternaires de vinylpyrrolidone et de vinylimidazole ;
(4) les polysaccharides quaternisés :
(5) les chitosanes ou leurs sels,
(6) et leurs mélanges.

22. Composition selon l'une quelconque des revendications 19 à 21, **caractérisée en ce que** le polymère cationique est choisi parmi les copolymères d'acrylamide et de diméthylaminoéthyl méthacrylate quaternisé au sulfate de diméthyle ou avec un halogénure de diméthyle, les copolymères d'acrylamide et de chlorure de méthacryloyloxyéthyltriméthyl-ammonium, le copolymère d'acrylamide et de méthosulfate de méthacryloyloxy-éthyltriméthylammonium, les copolymères vinylpyrrolidone / acrylate ou méthacrylate de dialkylamino-alkyle quaternisés, les terpolymères méthacrylate de diméthylaminoéthyle / vinylcaprolactame / vinylpyrrolidone, les terpolymères méthacrylate de diméthylaminopropyle / méthacrylamide / vinylpyrrolidone, les copolymères à base de vinylpyrrolidone et vinylcaprolactame, les polymères de chlorure de dimethyldiallyl ammonium, les copolymères d'acrylamide et de chlorure de diméthyldiallyl ammonium, les gommes de guar ou d'hydroxypropyl guar contenant des groupements cationiques trialkylammonium, les dérivés de cellulose quaternisés, et leurs mélanges.

23. Composition selon la revendication précédente, **caractérisée en ce que** le polymère cationique est choisi parmi les POLYQUATERNIUM 5, POLYQUATERNIUM 7, POLYQUATERNIUM 28, POLYQUATERNIUM 39, POLYQUATERNIUM 44, et leurs mélanges.

24. Composition selon l'une quelconque des revendications précédentes, **caractérisée en ce que** le polymère est un polymère amphotère choisi parmi les polymères comportant des motifs K et M répartis statistiquement dans la chaîne polymère, dans lesquels K et M peuvent avoir les significations suivantes :
• K désigne un motif dérivant d'un monomère comportant au moins un atome d'azote basique et M désigne un motif dérivant d'un monomère acide comportant un ou plusieurs groupements carboxyliques ou sulfoniques, ou bien
• K et M désignent des groupements dérivant de monomères zwitterioniques de carboxybétaïnes ou de sulfobétaïnes; ou bien
• K et M désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ; ou bien
• K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

25. Composition selon la revendication précédente, **caractérisée en ce que** le polymère amphotère est choisi parmi les dialkylaminoalkyl-méthacrylates et acrylates ; les dialkylaminoalkyl-méthacrylamides et acrylamides ; les copolymères d'acide acrylique et de chlorure de diéthyldiallylammonium ; et leurs mélanges.

26. Composition selon la revendication 24 ou 25, **caractérisée en ce que** le polymère amphotère est choisi parmi les polymères comportant des motifs dérivant :
a) d'au moins un monomère choisi parmi les acrylamides ou les méthacrylamides substitués sur l'azote par un radical alkyle comportant de 2 à 12 atomes de carbone,
b) d'au moins un comonomère acide contenant un ou plusieurs groupements carboxyliques réactifs, et
c) d'au moins un comonomère basique.

27. Composition selon l'une quelconque des revendications 24 à 26, **caractérisée en ce que** le polymère amphotère est choisi parmi les polyaminoamides réticulés et alcoylés ; les polymères comportant des motifs zwitterioniques de formule (V) : 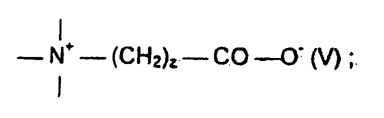 les polymères amphotères dérivés du chitosane ; les polymères amphotères dérivés de l'acide chloracétique ou du chloracétate de sodium ; les copolymères alkyl(C₁-C₅)-vinyléther / anhydride maléique modifié partiellement par semi-amidification avec une N,N-dialkylaminoalkylamine ; et leurs mélanges.

28. Composition selon l'une quelconque des revendications 24 à 27, **caractérisée en ce que** le polymère amphotère est le terpolymère acide acrylique / acrylate de méthyle / chlorure de méthacryl-amidopropyltrimonium ou Polyquaternium 47.

29. Composition selon l'une quelconque des revendications précédentes, **caractérisée en ce qu**'elle comprend en outre au moins un solvant choisi parmi les alcools comportant de 1 à 6 atomes de carbone, les polyols, et leurs mélanges.

30. Composition selon l'une quelconque des revendications précédentes, **caractérisée en ce qu**'elle comprend en outre au moins un actif choisi parmi les vitamines ou provitamines hydrosolubles ou liposolubles et leurs dérivés ; les stéroïdes ; les antiseptiques ; les antisébohrréïques ; les antimicrobiens ; les hydratants ; les agents kératolytiques et anti-âge ; les enzymes et co-enzymes ; les filtres solaires ; les azurants optiques ; les actifs amincissants ; les anti-inflammatoires ; et leurs mélanges.

31. Composition selon la revendication précédente, **caractérisée en ce que** l'actif est choisi parmi la vitamine A, la vitamine C, la vitamine B3, la vitamine B5, la vitamine E, la vitamine K1, le bêta-carotène, et leurs dérivés ; la DHEA et la 7α-hydroxy DHEA ; le peroxyde de benzoyle, l'acide salicylique, le triclosan, le triclocarban, l'acide azélaïque ; la glycérine, l'acide hyaluronique, le pyrrolidone carboxylique acide et ses sels, la sérine, le xylitol, le tréhalose, l'ectoïne, les céramides, l'urée ; l'acide glycolique, l'acide citrique, l'acide lactique, l'acide salicylique et ses dérivés ; le coenzyme Q10 ; l'acide 18 β glycyrrhétinique, l'acide ursolique, et leurs mélanges.

32. Composition selon l'une quelconque des revendications précédentes, **caractérisée en ce qu**'elle comprend au moins une charge.

33. Composition selon l'une quelconque des revendications 1 à 31, **caractérisée en ce qu**'elle constitue un masque.

34. Utilisation cosmétique de la composition selon l'une quelconque des revendications précédentes, comme produits de nettoyage et/ou de démaquillage de la peau, des yeux, du cuir chevelu et/ou des cheveux.

35. Utilisation de la composition selon l'une quelconque des revendications 1 à 33, pour la préparation d'une composition destinée à désinfecter la peau et/ou le cuir chevelu et contenant un antibactérien.

36. Procédé cosmétique de nettoyage de la peau, des yeux, du cuir chevelu et/ou des cheveux, **caractérisé en ce qu**'on applique la composition selon l'une quelconque des revendications 1 à 33, sur la peau, sur les yeux, sur le cuir chevelu et/ou sur les cheveux, en présence d'eau, et qu'on élimine la mousse formée par rinçage à l'eau.

## Patentansprüche

1. Zusammensetzung für die Reinigung, die in einem physiologisch akzeptablen, wässrigen Medium (1) mindestens einen schaumbildenden grenzflächenaktiven Stoff, (2) mindestens 1 Ges.-% mindestens einer Kieselsäure, bezogen auf das Gesamtgewicht der Zusammensetzung, (3) mindestens eine alkoxylierte Verbindung, die unter ethoxyliertem (120 EO) Methylglucosedioleat, ethoxyliertem Pentaerythrityltetrastearat (150 EO), dem Copolymer ethoxylierter (100 EO) Stearylalkohol/Polyethylenglycol (136 EO)/Hexamethyl-endiisocyanat und deren Gemischen ausgewählt ist, und (4) mindestens ein Polymer enthält, das unter den kationischen Polymeren, amphoteren Polymeren und deren Gemischen ausgewählt ist, wobei der Mengenanteil des schaumbildenden grenzflächenaktiven Stoffes/der schaumbildenden grenzflächenaktiven Stoffe im Bereich von 3 bis 50 Gew.-% wirksame Substanz, bezogen auf das Gesamtgewicht der Zusammensetzung, liegt.

2. Zusammensetzung nach Anspruch 1, **dadurch gekennzeichnet, dass** sie mindestens 35 Ges.-% Wasser, bezogen auf das Gesamtgewicht der Zusammensetzung, enthält.

3. Zusammensetzung nach Anspruch 1 oder 2, **dadurch gekennzeichnet, dass** sie 35 bis 95 Gew.-% Wasser, bezogen auf das Gesamtgewicht der Zusammensetzung, enthält.

4. Zusammensetzung nach einem der vorhergehenden Ansprüche, **dadurch gekennzeichnet, dass** sie eine Viskosität von 0,5 bis 25 Pa.s aufweist.

5. Zusammensetzung nach einem der vorhergehenden Ansprüche, **dadurch gekennzeichnet, dass** der Mengenanteil der Kieselsäure(n) im Bereich von 1 bis 15 Gew.-% wirksame Substanz, bezogen auf das Gesamtgewicht der Zusammensetzung, liegt.

6. Zusammensetzung nach einem der vorhergehenden Ansprüche, **dadurch gekennzeichnet, dass** die Kieselsäure unter den hydrophilen Kieselsäuren, hydrophoben Kieselsäuren und deren Gemischen ausgewählt ist.

7. Zusammensetzung nach dem vorhergehenden Anspruch, **dadurch gekennzeichnet, dass** die hydrophile Kieselsäure unter den Kieselsäuren pyrogener Herkunft oder Fällungskieselsäuren und deren Gemischen ausgewählt ist.

8. Zusammensetzung nach Anspruch 6 oder 7, **dadurch gekennzeichnet, dass** die hydrophile Kieselsäure unter den Kieselsäuren ausgewählt ist, die eine spezifische Oberfläche von 30 bis 500 m²/g, eine zahlenmittlere Größe der Partikel von 3 bis 50 nm und eine Stampfdichte von 40 bis 200 g/l aufweist.

9. Zusammensetzung nach einem der Ansprüche 6 bis 8, **dadurch gekennzeichnet, dass** die hydrophile Kieselsäure eine pyrogene Kieselsäure ist.

10. Zusammensetzung nach einem der Ansprüche 6 bis 8, **dadurch gekennzeichnet, dass** die hydrophile Kieselsäure aus einem umhüllten Partikel von hydrophiler Kieselsäure besteht.

11. Zusammensetzung nach einem der Ansprüche 6 bis 10, **dadurch gekennzeichnet, dass** die hydrophobe Kieselsäure unter den amorphen Kieselsäuren pyrogener Herkunft ausgewählt ist.

12. Zusammensetzung nach einem der Ansprüche 6 bis 11, **dadurch gekennzeichnet, dass** die hydrophobe Kieselsäure unter den Kieselsäuren mit einer spezifischen Oberfläche von 50 bis 500 m²/g, einer zahlenmittleren Partikelgröße von 3 bis 50 nm und einer Stampfdichte von 40 bis 200 g/l ausgewählt ist.

13. Zusammensetzung nach einem der vorhergehenden Ansprüche, **dadurch gekennzeichnet, dass** der Mengenanteil der alkoxylierten Verbindung(en) im Bereich von 0,1 bis 20 Gew.-% wirksame Substanz, bezogen auf das Gesamtgewicht der Zusammensetzung, liegt.

14. Zusammensetzung nach einem der vorhergehenden Ansprüche, **dadurch gekennzeichnet, dass** der schaumbildende grenzflächenaktive Stoff unter den nichtionischen grenzflächenaktiven Stoffen, anionischen grenzflächenaktiven Stoffen, amphoteren und zwitterionischen grenzflächenaktiven Stoffen und deren Gemischen ausgewählt ist.

15. Zusammensetzung nach einem der vorhergehenden Ansprüche, **dadurch gekennzeichnet, dass** der Mengenanteil des schaumbildenden grenzflächenaktiven Stoffes/der schaumbildenden grenzflächenaktiven Stoffe im Bereich von 3 bis 30 Gew.-% wirksame Substanz, bezogen auf das Gesamtgewicht der Zusammensetzung, liegt.

16. Zusammensetzung nach einem der vorhergehenden Ansprüche, **dadurch gekennzeichnet, dass** der schaumbildende grenzflächenaktive Stoff unter den Alkylpolyglucosiden, Maltoseestern, mehrfach mit Glycerin veretherten Fettalkoholen, Glucaminderivaten, Carboxylaten, Aminosäurederivaten, Alkylsulfaten, Alkylethersulfaten, Sulfonaten, Isethionaten, Tauraten, Sulfosuccinaten, Alkylsulfoacetaten, Phosphaten und Alkylphosphaten, Polypeptiden, anionischen Alkylpolyglucosidderivaten, Fettsäureseifen, Betainen, N-Alkylamidobetainen und deren Derivaten, Glycinderivaten, Sultainen, Alkylpolyaminocarboxylaten, Alkylamphoacetaten und deren Gemischen ausgewählt ist.

17. Zusammensetzung nach dem vorhergehenden Anspruch, **dadurch gekennzeichnet, dass** der schaumbildende grenzflächenaktive Stoff unter Decylglucosid, Caprylyl/Caprylglucosid, Laurylglucosid, Cocoglucosid, Laurylmonophosphat, dem Kaliumsalz von Dodecylphosphorsäure, dem Gemisch aus Octylphosphorsäuremonoester und Octylphosphorsäurediester, dem Kaliumsalz oder Triethanolaminsalz von Monoalkyl(C₁₂₋₁₃)phosphat, Kaliumlaurylphosphat und deren Gemischen ausgewählt ist.

18. Zusammensetzung nach einem der vorhergehenden Ansprüche, **dadurch gekennzeichnet, dass** der Mengenanteil des (der) kationischen und/oder amphoteren Polymers (Polymere) im Bereich von 0,01 bis 5 Gew.-% wirksame Substanz, bezogen auf das Gesamtgewicht der Zusammensetzung, liegt.

19. Zusammensetzung nach einem der vorhergehenden Ansprüche, **dadurch gekennzeichnet, dass** das Polymer ein kationisches Polymer mit einer Molmasse von 500 bis etwa 5 000 000 ist.

20. Zusammensetzung nach einem der vorhergehenden Ansprüche, **dadurch gekennzeichnet, dass** das Polymer ein kationisches Polymer mit einer Ladungsdichte von 0,9 bis 7 meq/g ist.

21. Zusammensetzung nach einem der vorhergehenden Ansprüche, **dadurch gekennzeichnet, dass** das Polymer ein kationisches Polymer ist, das ausgewählt ist unter:
(1) Homopolymeren oder Copolymeren, die ausgehend von einem oder mehreren ungesättigten Monomeren, die mindestens eine quartäre Ammoniumgruppe enthalten, einer der folgenden Formeln hergestellt werden: 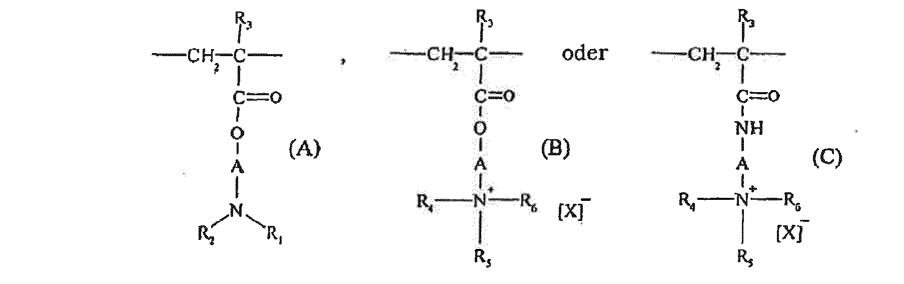 worin bedeuten:
R₃ ein Wasserstoffatom oder die Gruppe CH₃;
A eine geradkettige oder verzweigte Alkylengruppe mit 1 bis 6 Kohlenstoffatomen oder eine Hydroxyalkylengruppe mit 1 bis 4 Kohlenstoffatomen;
R₄, R₅ und R₆, die gleich oder verschieden sind,
eine Alkylgruppe mit 1 bis 18 Kohlenstoffatomen oder eine Benzylgruppe;
R₁ und R₂ ein Wasserstoffatom oder eine Alkylgruppe mit 1 bis 6 Kohlenstoffatomen;
X ein Methosulfatanion oder ein Halogenid wie Chlorid oder Bromid;
(2) Homopolymeren oder Copolymeren, die ausgehend von einem oder mehreren ungesättigten Monomeren hergestellt werden, die eine Dimethyldiallylammoniumgruppe aufweisen;
(3) quartären Copolymeren von Vinylpyrrolidon und Vinylimidazol;
(4) quaternisierten Polysacchariden;
(5) Chitosanen oder deren Salzen;
(6) und deren Gemischen.

22. Zusammensetzung nach einem der Ansprüche 19 bis 21, **dadurch gekennzeichnet, dass** das kationische Polymer ausgewählt ist unter: Copolymeren von Acrylamid und mit Dimethylsulfat oder einem Dimethylhalogenid quaternisiertem Dimethylaminoethylmethacrylat, Copolymeren von Acrylamid und Methacryloyloxyethyltrimethyl-ammoniumchlorid, dem Copolymer von Acrylamid und Methacryloyloxyethyltrimethylammoniummethosulfat, quaternisiertem Vinylpyrrolidon/Dialkylaminoalkylacrylat oder - methacrylat-Copolymeren, Dimethylaminoethylmethacrylat/Vinylcaprolactam/Vinylpyrrolidon-Terpolymeren, Dimethylaminopropylmethacrylat/Methacrylamid/Vinylpyrro lidon-Terpolymeren, Copolymeren auf der Basis von Vinylpyrrolidon und Vinylcaprolactam, Dimethyldiallylammoniumchlorid-Polymeren, Copolymeren von Acrylamid und Dimethyldiallylammoniumchlorid, Guargummen oder Hydroxypropylguar mit kationischen Trialkylammoniumgruppen, quaternisierten Cellulosederivaten und deren Gemischen ausgewählt ist.

23. Zusammensetzung nach dem vorhergehenden Anspruch, **dadurch gekennzeichnet, dass** das kationische Polymer unter POLYQUATERNIUM 5, POLYQUATERNIUM 7, POLYQUATERNIUM 28, POLYQUATERNIUM 39, POLYQUATERNIUM 44 und deren Gemischen ausgewählt ist.

24. Zusammensetzung nach einem der vorhergehenden Ansprüche, **dadurch gekennzeichnet, dass** das Polymer ein amphoteres Polymer ist, das unter den Polymeren ausgewählt ist, die statistisch in der Polymerkette verteilte Einheiten K und M enthalten, wobei K und M die folgenden Bedeutungen haben können:
• K bedeutet eine Einheit, die von einem Monomer abgeleitet ist, das mindestens ein basisches Stickstoffatom aufweist, und M bedeutet eine Einheit, das von einem Säuremonomer abgeleitet ist, das eine oder mehrere Carboxygruppen oder Sulfonsäuregruppen enthält, oder
• K und M bedeuten Gruppen, die von zwitterionischen Carboxybetainmonomeren oder Sulfobetainmonomeren abgeleitet sind; oder
• K und M bedeuten eine kationische Polymerkette, die primäre, sekundäre, tertiäre oder quartäre Aminogruppen aufweist, in der mindestens eine Aminogruppe eine Carbonsäuregruppe oder Sulfonsäuregruppe trägt, die über eine Kohlenwasserstoffgruppe gebunden ist; oder
• K und M sind Teil einer Polymerkette mit α, β-Dicarboxyethyleneinheit, bei der eine Carboxygruppe mit einem Polyamin umgesetzt wurde, das eine oder mehrere primäre oder sekundäre Aminogruppe aufweist.

25. Zusammensetzung nach dem vorhergehenden Anspruch, **dadurch gekennzeichnet, dass** das amphotere Polymer unter den Dialkylaminoalkyl-methacrylaten und - acrylaten; Dialkylaminoalkyl-methacrylamiden und - acrylamiden; Copolymeren von Acrylsäure und Diethyldiammoniumchlorid; und deren Gemischen ausgewählt ist.

26. Zusammensetzung nach Anspruch 24 oder 25, **dadurch gekennzeichnet, dass** das amphotere Polymer unter den Polymeren ausgewählt ist, die Einheiten enthalten, abgeleitet von:
a) mindestens einem Monomer, das unter den Acrylamiden oder Methacrylamiden ausgewählt ist, die am Stickstoff mit einer Alkylgruppe mit 2 bis 12 Kohlenstoffatomen substituiert sind,
b) mindestens einem sauren Comonomer, das eine oder mehrere reaktive Carboxygruppen aufweist, und
c) mindestens einem basischen Comonomer.

27. Zusammensetzung nach einem der Ansprüche 24 bis 26, **dadurch gekennzeichnet, dass** das amphotere Polymer unter den vernetzten und alkylierten Polyaminoamiden; Polymeren mit zwitterionischen Einheiten der Formel (V): 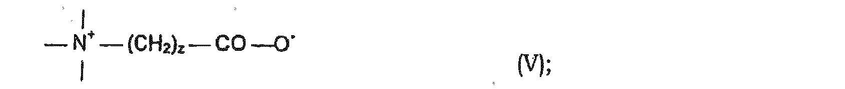 amphoteren, von Chitosan abgeleiteten Polymeren; amphoteren Polymeren, die von Chloressigsäure oder Natriumchloracetat abgeleitet sind, Copolymeren Alkyl(C₁₋₅)vinylether/teilweise durch Semiveresterung mit einem N,N-Dialkylaminoalkylamin modifiziertem Maleinsäureanhydrid; und deren Gemischen ausgewählt ist.

28. Zusammensetzung nach einem der Ansprüche 24 bis 27, **dadurch gekennzeichnet, dass** das amphotere Polymer ein Terpolymer Acrylsäure/Methylacrylat/Methacrylamidopropyltrimoniumchlorid oder Polyquaternium 47 ist.

29. Zusammensetzung nach einem der vorhergehenden Ansprüche, **dadurch gekennzeichnet, dass** sie ferner mindestens ein Lösungsmittel enthält, das unter den Alkoholen mit 1 bis 6 Kohlenstoffatomen, Polyolen und deren Gemischen ausgewählt ist.

30. Zusammensetzung nach einem der vorhergehenden Ansprüche, **dadurch gekennzeichnet, dass** sie ferner mindestens einen Wirkstoff enthält, der unter den wasserlöslichen oder fettlöslichen Vitaminen oder Provitaminen und deren Derivaten; Steroiden; antiseptischen Wirkstoffen; antiseborrhoischen Wirkstoffen; antimikrobiellen Wirkstoffen; Hydratisierungsmitteln; Keratolytika und Wirkstoffen gegen Alterung; Enzymen und Coenzymen; Sonnenschutzfiltern; optischen Aufhellern; schlanker machenden Wirkstoffen; entzündungshemmenden Wirkstoffen; und deren Gemischen ausgewählt ist.

31. Zusammensetzung nach dem vorhergehenden Anspruch, **dadurch gekennzeichnet, dass** der Wirkstoff unter Vitamin A, Vitamin C, Vitamin B3, Vitamin B5, Vitamin E, Vitamin K1, β-Carotin und deren Derivaten; DHEA und 7α-Hydroxy-DHEA; Benzoylperoxid, Salicylsäure, Triclosan, Triclocarban, Azelainsäure; Glycerin, Hyaluronsäure, Pyrrolidoncarbonsäure und ihren Salzen, Serin, Xylitol, Trehalose, Ectoin, Ceramiden, Harnstoff; Glycolsäure, Citronensäure, Milchsäure, Salicylsäure und ihren Derivaten; Coenzym Q10, 18β-Glycyrrhetinsäure, Ursolsäure und deren Gemischen ausgewählt ist.

32. Zusammensetzung nach einem der vorhergehenden Ansprüche, **dadurch gekennzeichnet, dass** sie mindestens einen Füllstoff enthält.

33. Zusammensetzung nach einem der Ansprüche 1 bis 31, **dadurch gekennzeichnet, dass** es sich um eine Maske handelt.

34. Kosmetische Verwendung der Zusammensetzung nach einem der vorhergehenden Ansprüche als Produkt für die Reinigung und/ oder zum Abschminken der Haut, der Augen, der Kopfhaut und/oder der Haare.

35. Verwendung der Zusammensetzung nach einem der Ansprüche 1 bis 33 für die Herstellung einer Zusammensetzung, die für die Desinfektion der Haut und/oder der Kopfhaut vorgesehen ist und einen antibakteriellen Wirkstoff enthält.

36. Kosmetisches Verfahren für die Reinigung der Haut, der Augen, der Kopfhaut und/oder der Haare, **dadurch gekennzeichnet, dass** eine Zusammensetzung nach einem der Ansprüche 1 bis 33 auf die Haut, die Augen, die Kopfhaut und/oder die Haare in Gegenwart von Wasser aufgebracht wird und der gebildete Schaum durch Spülen mit Wasser entfernt wird.

## Claims

1. Cleansing composition comprising, in a physiologically acceptable aqueous medium, (1) at least one foaming surfactant, (2) at least 1% by weight of at least one silica, with respect to the total weight of the composition, (3) at least one oxyalkylenated compound chosen from oxyethylenated (120 EO) methylglucose dioleate, oxyethylenated (150 EO) pentaerythrityl tetrastearate, the oxyethylenated (100 EO) stearyl alcohol/polyethylene glycol (136 EO)/hexamethylene diisocyanate copolymer, and their mixtures, and (4) at least one polymer chosen from cationic polymers, amphoteric polymers and their blends, the amount of foaming surfactant(s) ranging from 3 to 50% by weight of active material with respect to the total weight of the composition.

2. Composition according to Claim 1, **characterized in that** it comprises at least 35% by weight of water with respect to the total weight of the composition.

3. Composition according to Claim 1 or 2, **characterized in that** it comprises from 35 to 95% by weight of water with respect to the total weight of the composition.

4. Composition according to any one of the preceding claims, **characterized in that** it exhibits a viscosity ranging from 0.5 to 25 Pa·s.

5. Composition according to any one of the preceding claims, **characterized in that** the amount of silica(s) ranges from 1 to 15% by weight of active material with respect to the total weight of the composition.

6. Composition according to any one of the preceding claims, **characterized in that** the silica is chosen from hydrophilic silicas, hydrophobic silicas and their mixtures.

7. Composition according to the preceding claim, **characterized in that** the hydrophilic silica is chosen from silicas of pyrogenic origin or of precipitated origin and their mixtures.

8. Composition according to Claim 6 or 7, **characterized in that** the hydrophilic silica is chosen from silicas having a specific surface of 30 to 500 m²/g, a number-average particle size ranging from 3 to 50 nm and a tamped density ranging from 40 to 200 g/l.

9. Composition according to any one of Claims 6 to 8, **characterized in that** the hydrophilic silica is a pyrogenic silica.

10. Composition according to any one of Claims 6 to 8, **characterized in that** the hydrophilic silica consists of a particle coated with hydrophilic silica.

11. Composition according to any one of Claims 6 to 10, **characterized in that** the hydrophobic silica is chosen from amorphous silicas of pyrogenic origin.

12. Composition according to any one of Claims 6 to 11, **characterized in that** the hydrophobic silica is chosen from silicas having a specific surface of 50 to 500 m²/g, a number-average particle size ranging from 3 to 50 nm and a tamped density ranging from 40 to 200 g/l.

13. Composition according to any one of the preceding claims, **characterized in that** the amount of oxyalkylenated compound(s) ranges from 0.1 to 20% by weight of active material with respect to the total weight of the composition.

14. Composition according to any one of the preceding claims, **characterized in that** the foaming surfactant is chosen from nonionic surfactants, anionic surfactants, amphoteric and zwitterionic surfactants, and their mixtures.

15. Composition according to any one of the preceding claims, **characterized in that** the amount of foaming surfactant(s) ranges from 3 to 30% by weight of active material with respect to the total weight of the composition.

16. Composition according to any one of the preceding claims, **characterized in that** the foaming surfactant is chosen from alkylpolyglucosides, maltose esters, polyglycerolated fatty alcohols, glucamine derivatives, carboxylates, amino acid derivatives, alkyl sulfates, alkyl ether sulfates, sulfonates, isethionates, taurates, sulfosuccinates, alkyl sulfoacetates, phosphates and alkyl phosphates, polypeptides, anionic alkylpolyglucoside derivatives, fatty acid soaps, betaines, N-alkylamidobetaines and their derivatives, glycine derivatives, sultaines, alkyl polyaminocarboxylates, alkylamphoacetates, and their mixtures.

17. Composition according to the preceding claim, **characterized in that** the foaming surfactant is chosen from decylglucoside, caprylyl/caprylglucoside, laurylglucoside, cocoglucoside, lauryl monophosphate, the potassium salt of dodecyl phosphate, the mixture of octyl phosphate monoester and diester, the potassium or triethanolamine salt of mono (C₁₂-C₁₃) alkyl phosphate, potassium lauryl phosphate, and their mixtures.

18. Composition according to any one of the preceding claims, **characterized in that** the amount of cationic and/or amphoteric polymer(s) ranges from 0.01 to 5% by weight of active material with respect to the total weight of the composition.

19. Composition according to any one of the preceding claims, **characterized in that** the polymer is a cationic polymer having a molecular weight ranging from 500 to approximately 5 000 000.

20. Composition according to any one of the preceding claims, **characterized in that** the polymer is a cationic polymer having a charge density ranging from 0.9 to 7 meq/g.

21. Composition according to any one of the preceding claims, **characterized in that** the polymer is a cationic polymer chosen from:
(1) homopolymers or copolymers obtained from one or more unsaturated monomers and comprising at least one quaternary ammonium group, having one of the following formulae: 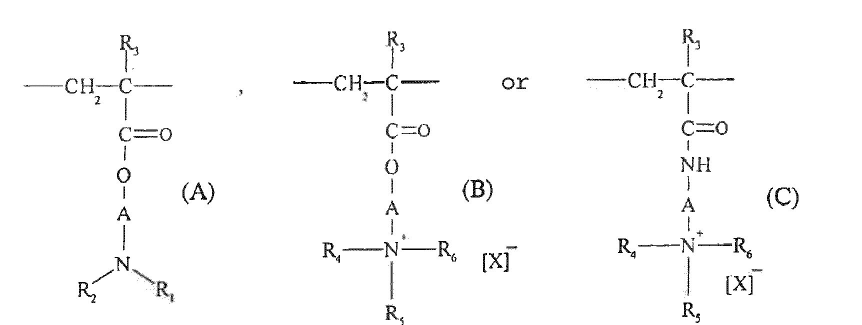 in which:
R₃ denotes a hydrogen atom or a CH₃ radical;
A is a linear or branched alkylene group comprising from 1 to 6 carbon atoms or a hydroxyalkylene group comprising from 1 to 4 carbon atoms;
R₄, R₅ and R₆, which are identical or different,
represent an alkyl group having from 1 to 18 carbon atoms or a benzyl radical;
R₁ and R₂ represent a hydrogen atom or an alkyl group having from 1 to 6 carbon atoms;
X denotes a methyl sulfate anion or a halide, such as chloride or bromide;
(2) homopolymers or copolymers obtained from one or more unsaturated monomers and comprising a dimethyldiallylammonium radical;
(3) quaternary copolymers of vinylpyrrolidone and of vinylimidazole;
(4) quaternized polysaccharides;
(5) chitosans or their salts;
(6) and their blends.

22. Composition according to any one of Claims 19 to 21, **characterized in that** the cationic polymer is chosen from copolymers of acrylamide and of dimethylaminoethyl methacrylate quaternized with dimethyl sulfate or with a dimethyl halide, copolymers of acrylamide and of methacryloyloxyethyltrimethylammonium chloride, the copolymer of acrylamide and of methacryloyloxyethyltrimethylammonium methyl sulfate, vinylpyrrolidone/dialkylaminoalkyl acrylate or methacrylate quaternized copolymers, dimethylaminoethyl methacrylate/vinylcaprolactam/vinylpyrrolidone terpolymers, dimethylaminopropyl methacrylate/methacrylamide/vinylpyrrolidone terpolymers, copolymers based on vinylpyrrolidone and vinylcaprolactam, dimethyldiallylammonium chloride polymers, copolymers of acrylamide and of dimethyldiallylammonium chloride, guar or hydroxypropyl guar gums comprising trialkylammonium cationic groups, quaternized cellulose derivatives, and their blends.

23. Composition according to the preceding claim, **characterized in that** the cationic polymer is chosen from Polyquaternium 5, Polyquaternium 7, Polyquaternium 28, Polyquaternium 39, Polyquaternium 44, and their blends.

24. Composition according to any one of the preceding claims, **characterized in that** the polymer is an amphoteric polymer chosen from polymers comprising K and M units distributed randomly in the polymer chain, in which polymers K and M can have the following meanings:
• K denotes a unit deriving from a monomer comprising at least one basic nitrogen atom and M denotes a unit deriving from an acidic monomer comprising one or more carboxylic or sulfonic groups; or else
• K and M denote groups deriving from carboxybetaine or sulfobetaine zwitterionic monomers; or else
• K and M denote a cationic polymer chain comprising primary, secondary, tertiary or quaternary amine groups, in which chain at least one of the amine groups carries a carboxylic or sulfonic group connected via a hydrocarbonaceous radical; or else
• K and M form part of a chain of a polymer comprising an α,β-dicarboxylic ethylene unit, one of the carboxylic groups of which has been reacted with a polyamine comprising one or more primary or secondary amine groups.

25. Composition according to the preceding claim, **characterized in that** the amphoteric polymer is chosen from dialkylaminoalkyl methacrylates and acrylates; dialkylaminoalkylmethacrylamides and dialkylaminoalkylacrylamides; copolymers of acrylic acid and of diethyldiallylammonium chloride; and their blends.

26. Composition according to Claim 24 or 25, **characterized in that** the amphoteric polymer is chosen from polymers comprising units deriving:
a) from at least one monomer chosen from acrylamides or methacrylamides substituted on the nitrogen by an alkyl radical comprising from 2 to 12 carbon atoms,
b) from at least one acidic comonomer comprising one or more reactive carboxylic groups, and
c) from at least one basic comonomer.

27. Composition according to any one of Claims 24 to 26, **characterized in that** the amphoteric polymer is chosen from crosslinked and alkylated polyaminoamides; polymers comprising zwitterionic units of formula (V): 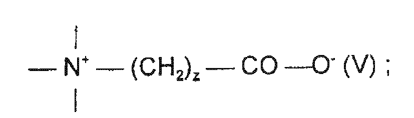 amphoteric polymers derived from chitosan; amphoteric polymers derived from chloroacetic acid or sodium chloroacetate; (C₁-C₅)alkyl vinyl ether/maleic anhydride copolymers, the maleic anhydride being partially modified by semiamidation with an N,N-dialkylaminoalkylamine; and their blends.

28. Composition according to any one of Claims 24 to 27, **characterized in that** the amphoteric polymer is the acrylic acid/methyl acrylate/methacryl-amidopropyltrimonium chloride terpolymer or Polyquaternium 47.

29. Composition according to any one of the preceding claims, **characterized in that** it additionally comprises at least one solvent chosen from alcohols comprising from 1 to 6 carbon atoms, polyols, and their mixtures.

30. Composition according to any one of the preceding claims, **characterized in that** it additionally comprises at least one active principle chosen from water-soluble or fat-soluble vitamins or provitamins and their derivatives; steroids; antiseptics; antiseborrhoeics; antimicrobials; moisturizing agents; keratolytic and antiageing agents; enzymes and coenzymes; sunscreens; optical brighteners; slimming active principles; antiinflammatories; and their mixtures.

31. Composition according to the preceding claim, **characterized in that** the active principle is chosen from vitamin A, vitamin C, vitamin B3, vitamin B5, vitamin E, vitamin K1, β-carotene, and their derivatives; DHEA and 7α-hydroxy-DHEA; benzoyl peroxide, salicylic acid, triclosan, triclocarban or azelaic acid; glycerol, hyaluronic acid, pyrrolidonecarboxylic acid and its salts, serine, xylitol, trehalose, ectoin, ceramides or urea; glycolic acid, citric acid, lactic acid, salicylic acid and its derivatives; coenzyme Q10; 18-β-glycyrrhetinic acid, ursolic acid, and their mixtures.

32. Composition according to any one of the preceding claims, **characterized in that** it comprises at least one filler.

33. Composition according to any one of Claims 1 to 31, **characterized in that** it constitutes a mask.

34. Cosmetic use of the composition according to any one of the preceding claims as products for cleansing and/or removing make-up from the skin, eyes, scalp and/or hair.

35. Use of the composition according to any one of Claims 1 to 33 in the preparation of a composition intended to disinfect the skin and/or the scalp and which comprises an antibacterial agent.

36. Cosmetic process for cleansing the skin, eyes, scalp and/or hair, **characterized in that** the composition according to any one of Claims 1 to 33 is applied to the skin, to the eyes, to the scalp and/or to the hair in the presence of water and **in that** the lather formed is removed by rinsing with water.
